(19) Europäisches Patentamt
European Patent Office
Office européen des brevets

(11) Publication number: 0 197 612
A2

# (12) EUROPEAN PATENT APPLICATION

(21) Application number: 86200580.8

(22) Date of filing: 04.04.86

(51) Int. Cl.⁴: C07C 85/00 , C07D 295/02

(30) Priority: 04.04.85 US 720150

(43) Date of publication of application: 15.10.86 Bulletin 86/42

(84) Designated Contracting States: BE DE FR GB IT NL SE

(71) Applicant: UNION CARBIDE CORPORATION
39 Old Ridgebury Road
Danbury Connecticut 06817(US)

(72) Inventor: Matherne, Joseph Lubin
1925 Massey Circle
So. Charleston West Virginia 25303(US)
Inventor: Doumaux, Arthur Roy, Jr.
1401 Wilkie Drive
Charleston West Virginia 25314(US)

(74) Representative: Smulders, Theodorus A.H.J. et al
Vereenigde Octrooibureaux Nieuwe Parklaan 107
NL-2587 BP 's-Gravenhage(NL)

## (54) Interchangeable process scheme.

(57) Process for utilizing a reaction zone containing a catalyst to interchangeably conduct separate organic amine condensation reactions and a separation zone to interchangeably conduct separations of the reaction product streams of such reactions. A first organic condensation reaction using a first reactant or set of reactants is conducted in the reaction zone in the presence of a catalytically effective amount of the catalyst and at a temperature and pressure sufficient for the first organic condensation reaction to occur. Then, after termination of the first organic condensation reaction, a second organic condensation reaction using a second reactant or set of reactants is conducted in the reaction zone in the presence of a catalytically effective amount of the catalyst and at a temperature and pressure sufficient for the second organic condensation reaction to occur. The reaction product from each of the separate organic reactions is separated into at least one component, e.g., reaction product, using the same separation zone. The reaction zone contains at least two distillation columns, preferably five distillation columns.

EP 0 197 612 A2

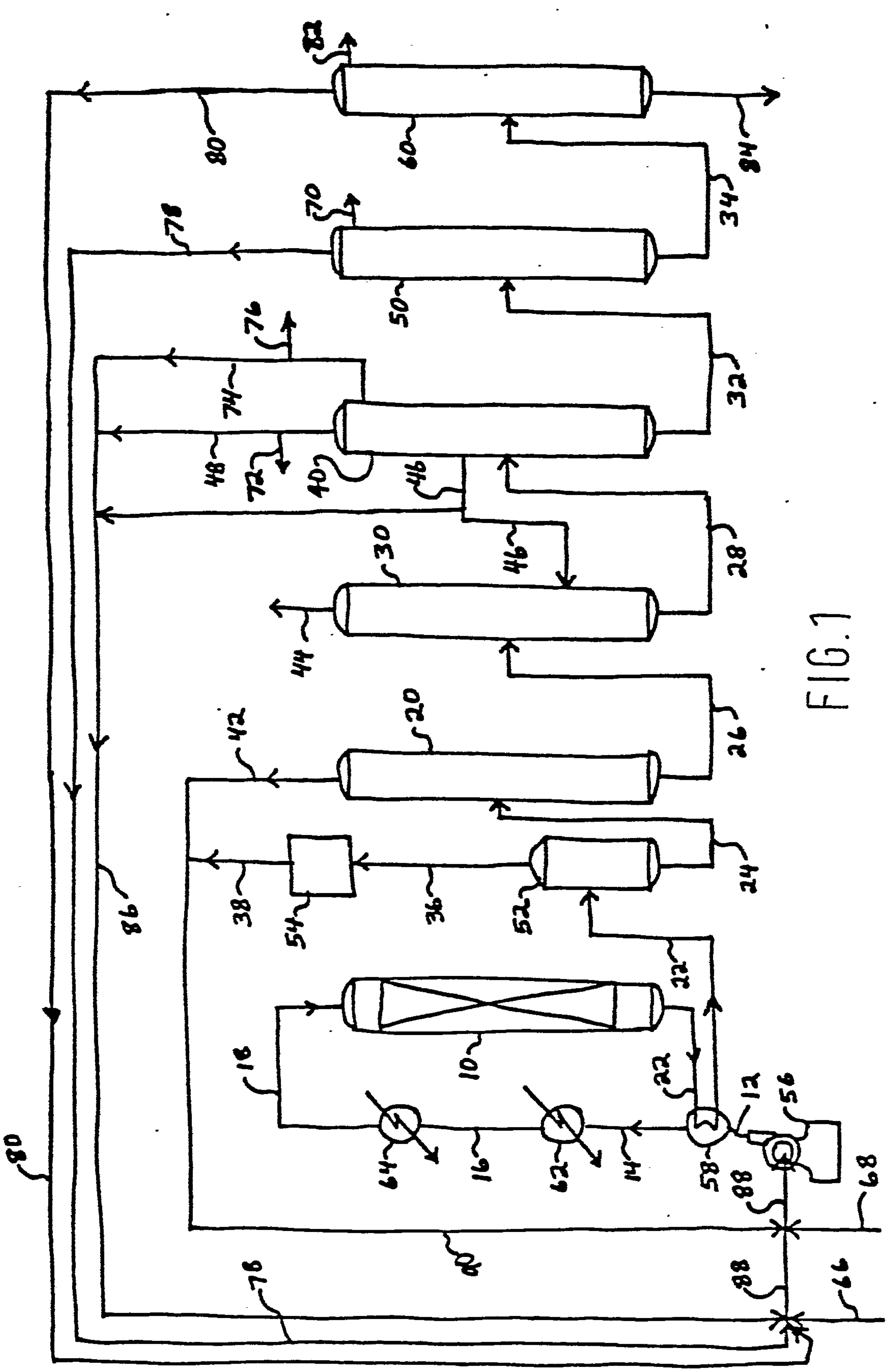
82
84
80
60
70
78
34
50
76
74
32
48
72
40
46
28
30
46
44
20
42
26
24
38
54
36
52
86
22
18
10
22
12
56
64
62
16
14
58
88
68
80
90
78
88
66

FIG. 1

INTERCHANGEABLE PROCESS SCHEME

## BACKGROUND OF THE INVENTION

The invention relates to the preparation of cyclic and noncyclic organic amines.

The chemical process industries usually design a set of equipment to produce one product or, at best, by adjusting reactant ratios, temperatures, etc., can adjust the mixture of products and by-products within a spectrum of products resulting from one type of reaction. Needless to say, such an approach is quite expensive as it requires heavy design and capital expenditures everytime a company wants to produce a new type of product - (which may even be very similar to another product being produced on the same plant site). Sometimes a company can convert a line of plant production equipment from producing a terminated chemical product to producing a different chemical product; still this does not eliminate the practice of a separate set of production equipment for each separate chemical product.

Organic synthesis by condensation reactions resulting in the loss of a molecule of water or of ammonia are well known in the art. Certain of such reactions are generally effected in the presence of acidic catalysts.

U.S. Patent No. 3,297,701 discloses a process for the production of diazabicyclo-(2.2.2)-octane and C-substi-tuted diazabicyclo-(2.2.2)-octanes by cyclizing a compound such as ethylenediamine, diethylenetriamine, triethylenetetramine or N-aminoethylpiperazine in the presence of aluminum, calcium or iron phosphate.

U.S. Patent No. 3,045,018 discloses a method of recovering products from a nitrogenous mixture consisting essentially of compounds resulting from a synthesis of triethylenediamine at an elevated temperature. The mixture includes 1,4-diazabicyclo-(2.2.2)-octane and alkyl pyrazines. U.S. Patent No. 3,128,275 teaches a method involving contacting N-aminoethylpiperazine with a cyclization catalyst at a temperature within the range of 200° to 500°C. to form a reaction product containing triethylenediamine, piperazine, N-alkylpiperazines and light components. The reaction product is fractionated to provide a fraction boiling within the range of 160° to 190°C. The fraction boiling with the range of 160° to 190°C. is cooled to form a slurry of crude triethylenediamine crystals contaminated with piperazine compounds in a mother liquor, the crude triethylenediamine crystals are recovered and dissolved in an inert organic solvent. The organic solution of crude triethylenediamine is treated at a temperature above 50°C. with an amount of acrylonitrile in a chemical excess of the piperazine compounds to thereby substantially, selectively, and quantitatively cyanoethylate the piperazine components and form cyanoethylpiperazine compounds. The solution is cooled to a temperature of less than 50°C. to selectively precipitate high purity triethylenediamine, which is recovered. U.S. Patent No. 2,120,525 teaches a method of preparing triethylenediamine hexahydrate which includes dissolving an impure triethylenediamine feed stock containing from about 95 to about 99 weight percent of triethylenediamine in from about 50 to about 80 weight percent of water at a temperature within the range of 0° to 40°C. to form a solution. The solution is cooled to a temperature of at least about 35°C. sufficient to form a precipitate of triethylenediamine hexahydrate.

U.S. Patent No. 4,463,193 discloses a process for preparing predominantly noncyclic polyalkylene polyamines. Ammonia or a primary or secondary amine is contacted with an alkanolamine compound having an amino group and a primary or secondary hydroxy group and an alkyleneamine compound having two amino groups in the presence of a catalytically effective amount of a Group IIIB metal acid phosphate. A temperature is used which is sufficient to effect a reaction among the ammonia or amine, the alkanolamine compound and the alkyleneamine compound under a pressure sufficient to maintain a substantial amount of the ammonia or amine in the reaction zone.

U.S. Patent No. 4,400,539 discloses a continuous process for the manufacture of ethylenediamine from the products of the reaction of ethylene oxide and ammonia. There is a continuous recycle stream of monoethanolamine to the products of the reaction of ethylene oxide and ammonia. The amination of such products to the reaction of ethylene oxide and ammonia combined with the monoethanolamine recycle provides a feed stream to the amination reaction zone which contains at least 70 weight percent of monoethanolamine as well as diethanolamine and triethanolamine. The moles of ammonia provided to the amination reaction exceeds the molar concentration of alcoholic hydroxyl groups present in the amination feed. The feed to the amination reactor contains at least a 5 percent increase in the concentration of monoethanolamine over the concentration of mono ethanolamine in the reaction product stream from the reaction of ethylene oxide and ammonia.

European (Published) Patent Application No. 0069322 teaches cyclization reactions such as in the conversion of hydroxyethylpiperazine to triethylenediamine and morpholine to dimethylaminoethylmorpholine using certain hydrogen phosphate and pyrophosphate catalysts.

British Patent No. 1,494,886 discloses a process for producing a morpholine compound having the formula:

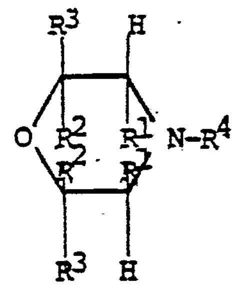

wherein $R^1$, $R^2$ and $R^3$, which may be the same or different, each is hydrogen or an alkyl radical, and $R^4$ is hydrogen or a substituted or unsubstituted alkyl, cycloalkyl, saturated heterocyclic or aryl radical. A glycol having the formula:

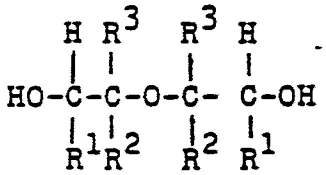

wherein $R^1$, $R^2$ and $R^3$ have the meanings given above, is contacted with ammonia or an amine having the formula:

$H_2N-R^4$

wherein $R^4$ has the meaning given above, in the presence of a phosphorus compound, at a temperature of from 220° to 350°C, under a pressure sufficient to maintain the reactants in the liquid phase.

U.S. Patent No. 4,501,889 discloses converting a diglycolamine compound to a morpholine compound at a temperature in the range of 285° to 420°C. in the presence of a catalyst, such as, the pyrophosphate, monohydrogen or dihydrogen phosphate or strontium.

U.S. Patent No. 4,254,060 discloses preparing aliphatic amines by reacting an aliphatic alcohol or an aliphatic aldehyde, with ammonia or a primary or secondary amine, in the presence of certain homogeneous colloidal catalysts.

U.S. Patent No. 4,210,605 discloses preparing aliphatic amines by reacting an aliphatic alcohol or an aliphatic aldehyde with ammonia or a primary or secondary aliphatic amine, in the presence of a homogeneous colloidal catalyst prepared by reducing a copper or silver salt of a carboxylic acid.

U.S. Patent No. 4,446,320 discloses using certain hydrogen phosphate and pyrophosphate compositions as catalysts for the organic condensation reactions of converting a diglycolamine compound to a morpholine compound.

U.S. Patent No. 4,233,447 discloses a process for directly recovering a substantially pure triethylenediamine solute in a 1,4-butanediol solution from a crude aqueous triethylenediamine reaction product mixture obtained from a liquid phase process for the preparation of triethylenediamine. The 1,4-butanediol is admixed with the crude triethylenediamine reaction product mixture to form a crude triethylenediamine reaction product-1,4-butanediol admixture. The admixture is heated under conditions such that water and other lower

boiling impurities are removed. The resultant bottoms are heated to produce a codistillate of a substantially pure triethylenediamine solute in liquid 1,4-butanediol solution.

U.S. Patent No. 3,993,651 also discloses a process for directly recovering a substantially pure triethylenediamine solute in liquid propylene glycol solution from a crude triethylenediamine reaction product mixture obtained from a liquid phase process for the preparation of triethylenediamine. Propylene glycol is admixed with the crude triethylenediamine reaction product mixture to form a crude triethylenediamine reaction product-propylene glycol admixture. This admixture is distilled under conditions such that the triethylenediamine and the propylene glycol codistillate. The resultant codistillate is collected as the substantially pure triethylenediamine solute in liquid propylene glycol solution.

## BROAD DESCRIPTION OF THE INVENTION

The invention involves a process for utilizing in combination a reaction zone containing a catalyst to interchangeably conduct separate organic amine condensation reactions and a separation zone to interchangeably conduct separations of the reaction product streams of such reactions. A first organic condensation reaction using a first reactant or set of reactants is conducted in the reaction zone in the presence of a catalytically effective amount of the catalyst and at a temperature sufficient for the first organic condensation reaction to occur. All or part of the first reaction product stream is separated in the separation zone into at least one constituent. The separation zone has at least two distillation columns, usually at least four (say up to seven or eight distillation columns) and preferably five distillation columns. The term constituent is used herein to encompass a compound, an azeotrope or stream containing at least two compounds or an azeotrope and at least one other compound, etc. Then, after termination of the first organic condensation, a second organic condensation reaction using a second reactant or set of reactants is conducted in the reaction zone in the presence of the catalyst and at a temperature and pressure sufficient for the second organic condensation reaction to occur. All or part of the second reaction product stream, after termination of the separation of the first reaction product stream, is separated in the separation zone into at least one constituent. The phrase "all or part of" is usually used herein to mean at least one weight percent, preferably at least 10 weight percent.

Any number of different types of organic amine condensation reactions can be conducted in the single reaction zone. The separate types of reactions can be sequentially conducted in any sequence as desired (for example, to produce products as required by sales requirements at the time). Reaction types can be alternated, repeated followed by alternation, etc. The catalyst can also be changed between reactions to a different catalyst (type), as desired.

Polyalkylene polyamines can be prepared by reacting an alkylenediamine having at least two amino groups (e.g., ethylenediamine) and an alkanolamine having at least one amino group (e.g. ethanolamine). Noncyclic polyalkylene polyamines, such as, diethylenetriamine, and cyclic polyalkylene polyamines, such as, piperazine, can be prepared.

Diazabicyclo-(2.2.2)-octane or C-substituted diazabicyclo-(2.2.2)-octanes can be prepared by the catalytic reaction of a compound having the formula:

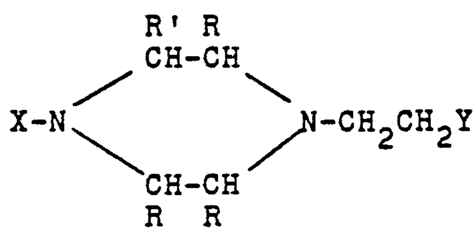

or a compound having the formula:

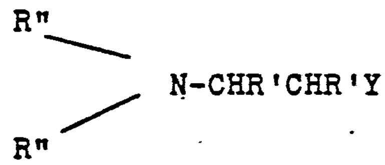

wherein R is hydrogen, an alkyl group containing 1 to 12 carbon atoms or a cycloalkyl group containing 6 to 12 carbon atoms, R' is hydrogen or an alkyl group con taining 1 to 4 carbon atoms, R" is hydrogen or -CHR'CHR'Y, X is hydrogen or -$CH_2CH_2Y$, and Y is -OH or -$NH_2$. For example, triethylenediamine can be prepared from N-aminoethylpiperazine.

Ethylenediamine can be prepared from ammonia and monoalkanolamine and can be prepared from a stream containing ammonia, monoethanolamine, diethanolamine and triethanolamine as produced by the direct reaction of ethylene oxide and ammonia.

Aliphatic amines, such as, alkyl amines, can be prepared by reacting an aliphatic alcohol, such as, an alkanol, and an aliphatic aldehyde, with ammonia or a primary amine or a secondary amine.

Morpholine and morpholine compounds can be prepared from diglycolamine and diglycolamine compounds, respectively.

The reaction is preferably conducted in the vapor phase or the supercritical phase, but can be conducted in the liquid phase. For purposes herein, sometimes, the term gas phase encompasses both the vapor phase and supercritical phase. Preferably the liquid hourly space velocity of the reactants in the reaction is between about 1 and about 25 per hour of the reactants. Preferably the catalyst used is an amination catalyst. One group of preferred phosphorus-containing catalysts is the Group IIIB metal phosphates, Group IIIB metal monohydrogen phosphates and Group IIIB metal dihydrogen phosphates supported on a carrier. Somewhat higher reaction temperatures are used with the Group IIIB metal acid phosphate catalysts. The amination catalyst can also be a reductive amination catalyst, preferably a nickel catalyst impregnated on a support material, such as, alumina, silica, silica-alumina, diatomaceous earth or silica-titania. The reaction is normally conducted at atmospheric or elevated pressure. The pressure in the reaction zone is most preferably 200 to 2,000 p.s.i.g., depending on the reaction being conducted and the catalyst being used. A gaseous diluent can be used to assist attaining and maintaining the desired elevated reaction pressure. Generally, the reaction temperatures are within the range of 125° to 425°C. Depending upon the reaction being conducted, the reaction is preferably between 150° and 235°C. when a reductive amination catalyst is used and is preferably between 220° and 350°C. when a phosphorus-containing catalyst is used.

The first reaction product stream is separated in separation zone into at least one constituent, e.g., a reaction product. After the first reaction has been terminated and the second reaction has been started, the reaction product stream from the second reaction is separated in the same separation zone into at least one constituent, e.g., a reaction product. The separation schemes used can be any conventional separation unit composed of at least two distillation columns (stills), flashing off unit and the like, which finds use in any of the individual organic amine condensation reaction processes to separate the reaction products, unreacted feed components, etc. Preferably the distillation columns are fractional distillation columns.

The invention also involves an embodiment composed of a process for utilizing a separation zone to interchangeably conduct separate separations of reaction product streams from separate organic amine condensation reactions. The separation zone has at least two distillation columns, preferably five distillation columns. A first separation is conducted in the separation zone of a first reaction product stream from a first organic amine condensation reaction into at least one constituent. After termination of the first separation, a second separation is conducted in the separation zone of a second reaction product stream from a second organic amine condensation reaction into at least one constituent. The separation schemes used can be any conventional separation unit composed of at least two distillation columns (stills), flashing off unit and the like, which finds use in any of the indivudal organic amine condensation reaction processes to separate the reaction products, unreacted feed components, etc.

An important advantage of the invention process is that it can be run in one set of equipment, even a set of existing plant process equipment. Without incurring the cost of multiple sets of process equipment, the invention scheme allows a

number of different reactions to be sequentially run in the same equipment without even having to change the catalyst. The construction material for the equipment used needs to be suitable for all of the reactions run therein.

BRIEF DESCRIPTION OF THE DRAWING

In the drawing:

Figure 1 is a schematic diagram of a preferred embodiment of the process of the invention; and

Figure 2 is a partial schematic diagram of one embodiment of the process of the invention.

DETAILED DESCRIPTION OF THE INVENTION

As used herein, all parts, percentages, ratios and proportions are on a weight basis and all temperatures are in degrees Centigrade, unless otherwise stated or otherwise obvious herefrom. Also as used herein, the term "ethanolamine" specifically means monoethanolamine unless otherwise indicated or implied herein. U.S. Sieve Series mesh sizes are used herein.

Basically, the equipment used in the invention process is a reaction zone followed by a separation zone.

In Figure 1, feed components are fed into the system via lines 66 and 68 and proceed to pump 56 via line 88. The pressurized feed is passed via lines 12, 14, 16 and 18 into the top of reactor 10. In transit pressurized feed passes through heat recovery exchanger 58, steam heat exchanger 62 and gas-fired heat exchanger 64. Reactor 10 contains a fixed bed of phosphorus-containing catalyst supported on inert catalyst support or a reductive amination catalyst (e.g., Ni) supported on an inert catalyst support. The reaction product stream - (effluent) is passed via line 22 through heat recovery exchanger 58 and on into ammonia flash tank 52 for the purpose of separating ammonia and any diluent from the liquid reaction product stream. - (Since hydrogen is used in the feed when a reductive amination catalyst is used, hydrogen will be separated along with the ammonia and any diluent by flash tank 52.) The ammonia is taken off through line 36 to condenser 54 -the condensed ammonia is recycled to reactor feed line 88 via lines 38 and 90. The bottoms from flash tank 52 are fed via line 24 into ammonia stripping still 20. The ammonia coming off of the top of still 20 is also recycled via lines 42 and 90. The bottoms from ammonia still 20 are passed via line 26 into distillation still 30 maintained at a temperature sufficient to remove the water contained in the reaction through line 44. The heavies from the reaction are recovered from distillation column 30 through line 28 and are passed to still 40 for the recovery of one component by way of line 48. The recovered component can be withdrawn via line 72 or fed via lines 48 and 86 into reactor feed line 88. Another higher boiling component can come off below the top of distillation column 40 and be withdrawn via lines 74 and 76 or fed via lines 74 and 86 into reactor feed line 88. The heavies from the distillation are removed through line 32 into distillation column (still) 50 from which one component is recovered through line 70 or fed via line 78 into reactor feed line 88. The bottoms from that separation are removed through line 34 and passed to distillation column - (still) 60 for the recovery of one component which is recycled by way of line 80 to reactor feed line 88 or is withdrawn via line 82. The bottoms of that separation can be passed from line 84 into a batch still or a series of refining columns for recovering each of the various remaining components of the stream.

By reaction zone is meant that vessel, e.g., autoclave, continuous stirred tank reactor or packed bed reactor, in which the catalyst is located and the production of polyalkylene polyamines is effected.

Although the reactions can be carried out in the batch mode, they are preferably conducted as continuous processes through a bed of particulate catalyst, for example, operation of a continuously stirred tank reactor or a packed (fixed) bed reactor. The continuous process is carried out by employing conventional process techniques and apparatus well known to those skilled in the art. In the continuous reaction processes, the catalyst can be added alone or in combination with the reactants, or, as stated above, the catalyst can be provided as a fixed bed on conventional support materials well known to those skilled in the art. The reaction is allowed to proceed until a desired conversion is obtained or the reaction is complete. Normally the reaction is carried out within about 0.5 to 5 hours in the batch mode or residence times (based on the ethylenediamine and ethanolamine components) of 0.1 to 4.0 hours in a continuous mode for practical levels of organic amine condensation product production.

The reactor can be an up-flow or down-flow reactor and can have a fluidized bed or, most commonly, a fixed bed. The catalyst bed can contain inert particles which can be interspersed throughout the bed and/or form discrete layers, e.g., at an end or intermediary to the bed. The volume of the reaction zone containing such inert particles is the reaction zone volume for purposes

of determining the feed rate. Preferably, the space velocity should not be so high that for the reactor geometry, a significant amount of backmixing occurs. Advantageously, the flow through the catalyst bed is substantially plug-type flow.

The mole ratio of reactants fed to the reaction zone depends upon the individual organic amine condensation reaction.

The reaction is preferably conducted in the vapor phase or the supercritical phase, although the reaction can be conducted in the liquid phase. As used herein, the phrase "gas phase" encompasses the gas phase and the supercritical phase. The liquid hourly space velocity of the reactants is between about 0.1 and about 100 per hour and preferably is between about 1 and about 25 per hour.

The reaction is usually conducted at atmospheric or elevated pressure. The pressure at the time of reaction should normally be within the range from about 1 to about 4,000 p.s.i., preferably greater than 100 p.s.i.g. and most preferably from about 200 to about 2,000 p.s.i.g. In any event, the pressure should be selected such that the reaction feedstream is maintained in the desired phase. Normally, a gaseous diluent, such as, hydrogen, methane, helium, water, nitrogen, argon and stoichiometrically-excessive ammonia, can be added to increase the pressure in the reactor.

In the reactor, the temperature for the reaction depends upon the particular starting materials, ratios of reactants, and most importantly, the activity of the catalyst used. Generally, in the process of the invention, temperatures within the range of 125° to 425°C. are suitable, but the temperature range actually used will depend upon the type of organic amine condensation reaction being run. When a reductive amination catalyst is used, preferably the reaction temperature range is 150° to 235°C. When a phosphorus-containing catalyst is used, the reaction temperature is preferably 220° to 350°C. and most preferably 260° to 300°C. A diluent gas can be utilized to help in the control of the reaction temperature and to assist in maintaining the desired pressure.

The catalysts used in the invention are heterogeneous catalysts. The catalysts are used in an amount of 0.1 to 12 weight percent, preferably 0.5 to 10 weight percent, and most preferably 2 to 7 weight percent, based on the total weight of the reactants.

The catalyst is preferably an amination catalyst, such as, reductive amination catalysts and phosphorus-containing catalysts.

Any suitable or conventional reductive amination catalyst can be used in the reactor. When a reductive amination catalyst is used, the reactor feed can include hydrogen. Preferably 4 to 12 mole percent, typically about 2 to 20 mole percent, based on the total moles of one of the reactants, preferably the most prevalent nitrogen-containing reactant, e.g., ammonia, of hydrogen is used.

Reductive amination catalysts are well-known in the art and usually comprise the metal or oxide of one or more of nickel, copper, cobalt, iron and the like as the active species. Other compounds which can find use in such catalysts include the metal, oxide or salt of one or more of chromium, lanthanum, lithium, potassium, cesium, cerium, ruthenium, rhodium, palladium, platinum, rhenium, iridium, silver, zinc, titanium, manganese and boron. Often the catalysts are the Raney nickel-type or Raney cobalt-type, or are supported catalysts. Many of the catalysts ae preferably activated at elevated temperature in a hydrogen atmosphere. Particularly desirable catalysts comprise nickel as the catalytically-active species.

The preferred reductive amination catalysts are those which are nickel on a catalyst support material and the most preferred are those which are a mixture of nickel and rhenium impregnated on various support materials including alumina, silica, silica-alumina, kieselguhr or diatomaceous earth and silica-titania. Preferably the mole ratio of the nickel to the rhenium is in the range of from 2:1 to about 30:1 and the total nickel and rhenium metal present is in the range of 3 to 30 percent by weight of the support. Such catalysts can be prepared by the methods taught in U.S. Patent Nos. 4,111,840 and 4,123,462, the pertinent parts of which are incorporated herein by reference. Basically, such catalysts are solid catalysts wherein the nickel and rhenium metals are supported on certain catalyst support materials.

Hydrogen is used in the reactor to maintain the activity of the reductive amination catalyst.

The nickel-rhenium catalysts can contain various other metals in admixture with the nickel and rhenium which do not detrimentally affect the catalytic properties of catalysts containing nickel and rhenium as the only impregnated metals. These additional metals, in certain amination processes, can actually improve selectivity and activity of the basic Ni-Re catalyst. Certain of these metals can extend the activity life and other physical properties of the Ni-Re catalyst. Examples of catalysts containing additional metal components include Ni-Re-La, Ni-Re-Ca, Ni-Re-Mg, Ni-Re-Sr, Ni-Re-Li, Ni-Re-K, Ni-Re-Ba, Ni-Re-Ce, Ni-Re-W, Ni-Re-Fe, Ni-Re-Ru, Ni-Re-Cu, Ni-Re-Ag, Ni-Re-Zn, Ni-Re-Co, Ni-Re-U, Ni-Re-Ti and Ni-Re-Mn.

The amount of Ni-Re catalyst present in the process depends on many variables including the reactants, the relative proportions of the reactants, reaction condition and the degree of conversion and selectivity desired. Moreover, the amount of catalyst will depend also on the nature of the catalyst itself, e.g., its metal loading and activity and age. The catalyst must be present in the reaction zone in sufficient catalytic amount to enable the desired reaction to occur. This is also true for any of the catalysts useful in the invention process.

Other preferred reductive amination catalysts are catalysts composed of rhenium, nickel and boron impregnated on a support material selected from the group consisting of aluminas (e.g., alpha), silicas, silica-aluminas, kieselguhrs or diatomaceous earths and silica-titanias, wherein the ratio of nickel to boron to rhenium is in the range of from about 2:2:1 to about 30:30:1 and the total nickel, boron and rhenium present is in the range of about 3 to about 30 percent by weight of the support material.

The most preferred mode of the catalyst is a reductive amination catalyst which has a silica support marketed under the mark T-869® by United Catalyst Incorporated. The best mode of the catalyst is obtained from the United Catalyst Incorporated. It is believed that the catalyst is prepared by impregnating the T-869® catalyst support with an aqueous solution containing nickel nitrate, ammonium perrhenate and boric acid in a metal composition of 66 percent of nickel, 19.6 percent of rhenium and 14.4 percent of boron. Following the impregnation, it is calcined at 330°C. for 2.5 hours. Then it is impregnated a second time with the same solution and calcined again under the same conditions for the same length of time. Thereafter it is impregnated a third time to reach a 12 percent total metal loading. It is then calcined at 330°C. for 2.5 hours. Then it is reduced under a hydrogen atmosphere at a temperature of 332° to 337°C. for 16 hours. Following the reduction step it is stablized at 60°C. for 30 to 40 hours.

Examples of other useful reductive amination catalysts are the rhodium atom-containing catalysts of U.S. Patent No. 4,322,530, the copper-rhenium catalyst of U.S. Patent No. 4,206,149, the nickel-cobalt-iron catalysts of U.S. Patent No. 3,766,184, the cobalt-nickel-copper-containing aluminum oxide or silicon dioxide support catalyst of U.S. Patent No. 4,014,933 and the catalysts containing copper oxide or copper hydroxide, nickel oxide or nickel hydroxide, and, optionally, an oxide or a hydroxide of a Group IIA metal of U.S. Patent No. 4,409,339 - the pertinent parts of such patents are incorporated herein by reference. U.S. Patent No. 4,209,424 (the pertinent parts of which are incorporated herein by reference) teaches an amination catalyst which has at least one active metal from the group of transition metals consisting of nickel, cobalt and copper, uniformly combined with a refractory microporous substance.

The pertinent parts of copending, commonly-assigned U.S. Patent Application Serial No. 454,485, filed on December 29, 1982, are incorporated herein by reference. U.S. Ser. No. 454,485 discloses a process for the production of an amine composition having a high ratio of diethylenetriamine to piperazine which comprises maintaining ethylenediamine in the presence of a nickel, cobalt or rhodium catalyst, wherein the metal is present on the surface of the catalyst in a polyatomic form, and at a temperature between about 170° to about 210°C. sufficient to convert less than about 35 percent of the ethylenediamine to polyamine. The catalyst of U.S. Ser. No. 454,485 are nickel, cobalt or rhodium catalysts which can be relatively pure metal catalysts or catalysts that have been modified by the addition of molybdenum, chromium, iron or other transition metals in varying amounts. The catalysts can be in a massive form or they can be supported on a carrier such as the preferred silica or alumina carriers wherein the metal is present on the surface of the catalyst in a polyatomic form. Preferred catalysts are Raney nickel or Raney cobalt or a Ni/Re/B on silica catalyst prepared as described in U.S. Patent No. 4,123,462. The catalyst charge, as a weight percent of the total charge, is not narrowly critical, although a charge of about 3 weight percent is preferred for the reaction temperature and times taught in U.S. Ser. No. 454,485. The reaction conditions and catalysts of U.S. Ser. No. 454,485 can be used in the reaction zone of the invention.

The pertinent portions of copending, commonly-assigned U.S. Patent Application Serial No. 613,116, filed on May 23, 1984, are incorporated herein by reference. U.S. Ser. No. 613,116 discloses reductive amination catalysts which are catalysts comprising a support material selected from the group consisting of alumina, silica, silica-alumina, kieselguhr, diatomaceous earth and silica-titania, and nickel and at least one potentiating agent selected from the group consisting of platinum and iridium wherein the catalyst has a total nickel and potentiating agent is in the range from about 1:1 to about 30:1. The potentiated nickel

catalyst includes catalysts which contain various other metals in admixture with the nickel and potentiating agent which do not detrimentally affect catalytic properties. These additional metals, in certain amination processes, may actually improve selectivity and activity of the potentiated nickel catalyst. Certain of these metals may extend the activity life and other physical properties of the catalyst. Examples of additional metal components include lanthanum, boron, magnesium, lithium, potassium, cesium, cerium, iron, ruthenium, copper, silver, zinc cobalt, palladium, titanium, manganese, rhodium, and rhenium. The amount of such additional metal components, ratioed to nickel and expressed as an atomic ration, is about 0.001:1 to 1:1, frequently about 0.01:1 to 0.5:1. Particularly preferred catalysts comprise nickel, iridium and/or rhenium. In these catalysts, the rhenium or iridium is generally provided in an atomic ratio to nickel of about 10:1 to 1:10.

When a reductive amination catalyst is used, the reaction temperature preferably is 150° to 235°C. and most preferably the reaction pressure is 200 to 2,000 p.s.i.g.

Any phosphorus-containing catalyst can be used. One group of preferred phosphorus-containing catalysts is the metal phosphate catalysts, which can be metal phosphates, metal monohydrogen phosphates, metal dihydrogen phosphates and metal pyrophosphates.

The metal phosphate catalysts include boron phosphate, aluminum phosphate, ferric phosphate, zinc phosphate, ferrous phosphate, nickel phosphate, chromium phosphate, copper phosphate and cobalt phosphate. Other metal phosphate catalysts which can be used are the phosphates of lithium, sodium, potassium, other metals of Group IA of the periodic tables, beryllium, magnesium, calcium, other metals of Group IIA of the periodic tables, titanium, zirconium, other metals of Group IVB of the periodic table, antimony and tin (valence states II and IV). Further useful catlysts are those which comprise a phosphorus bonded to a Group IVB transition metal oxide support, such as are disclosed in published European Patent Application 0115138 (the pertinent parts of which are incorporated herein by reference). Mixtures of two or more of the metal phosphate catalysts can be used.

The metal phosphate catalysts also include the pyrophosphates, monohydrogen phosphates and dihydrogen phosphates of strontium, copper, magnesium, calcium, barium, zinc, aluminum, cobalt, nickel, cerium, neodymium, and mixtures thereof. Specific examples of such catalysts are $SrHPO_4$, $Sr/BaHPO_4$, $Sr(H_2PO_4)_2$, $Ca(H_2PO_4)_2$, $Nd_2(HPO_4)_3$, $Ce_2(HPO_4)_3$, $CoHPO_4$, $NiHPO_4$, $Al_2(HPO_4)_3$, $MgHPO_4$, $BaHPO_4$, $CuHPO_4$ and $ZnHPO_4$.

The metal phosphate catalysts include the crystalline zirconium phosphates of U.S. Patent No. 3,416,884 and the granular zirconium phosphates of U.S. Patent No. 4,025,608 -the pertinent parts of such patents are incorporated herein by reference.

The metal phosphate catalysts which are most preferred for practicing the process of the invention are Group IIIB metal acid phosphates including Group IIIB metal phosphates, Group IIIB metal monohydrogen phosphates, Group IIIB metal dihydrogen phosphates and mixtures thereof. U.S. Patent No. 4,463,193 (the pertinent parts of the patent are incorporated herein by reference) discloses processes for preparing the Group IIIB metal acid phosphates. While the intent of the catalyst preparation is to specifically provide a particular Group IIIB monohydrogen phosphate or dihydrogen phosphate, mixtures of the Group IIB metal phosphates of the above-mentioned types can be obtained owing to complicated dependence of the catalyst composition on preparation conditions. Nevertheless, although the Group IIIB metal acid phosphate catalyst of the invention comprises the metal phosphate, monohydrogen phosphate, dihydrogen phosphate or mixtures thereof, the monohydrogen and dihydrogen phosphates of the Group IIIB metals are the preferred catalysts when in relatively pure form individually or in combination.

A Group IIIB metal is meant to include scandium, yttrium, lanthanum and the rare earth lanthanide metals having atomic numbers 58 to 71, and the rare earth actinides having atomic numbers 89 to 92.

The most preferred metal phosphate catalysts for the production of noncyclic polyalkylene polyamines are the acid phosphates, preferably the monohydrogen phosphates and dihydrogen phosphates, of scandium, lanthanum, cerium, samarium, europium, thulium, erbium, ytterbium, yttrium, luetium, thorium, neodymin, praseodymium, dysprosium and gadolinium.

The acid phosphate catalysts can be used for the production of polyalkylene polyamines either singly or in combination.

It is preferred to use those which are more catalytically active and provide for substantial conversion to the noncyclic polyalkylene polyamine products. Examples of the most preferred catalyst compounds include lanthanum monohydrogen phosphate, lanthanum dihydrogen phosphate, lanthanum phosphate, praseodymium monohydrogen

phosphate, praseodymium dihydrogen phosphate, praseodymium phosphates, neodymium monohydrogen phosphate, neodymium dihydrogen phosphate, neodymium phosphate and mixtures thereof.

The quantity of the acid phosphate salts of the Group IIIB metals used in the reaction can vary widely depending upon the reactivity of the catalysts and the reactivity of the reactants present. A catalytically effective amount of material is used; for example, an amount which causes a reaction of the ethylenediamine and ethanolamine to yield polyalkylene polyamine products at the temperature and pressure used. Usually though, the amount used to provide a catalytic effect ranges from about 0.1 to 25 mole percent based upon the total amount of reactants present in the reaction mixture, and preferably is an amount of about 0.1 to 10 mole percent. Within these ranges though, the level of catalyst is empirical and is adjusted depending on the product slate desired.

The Group IIIB metal phosphate catalysts used in the process of the invention can be prepared by the precipitation of the desired metal acid phosphate salt, washing the salt to remove inorganic coproducts and drying the salt. Optionally, dried catalysts can be further processed prior to use.

Such processing is well-known to those skilled in the art and includes extrusion or pelleting or compounding with an inert support such as alpha-alumina.

Methods of preparing Group IIIB metal monohydrogen phosphate or dihydrogen phosphate are disclosed in U.S. Patent No. 4,324,917 (the pertinent parts of the patent are incorporated herein by reference). Phosphate-containing materials can be obtained which consist predominantly of the Group IIIB metal phosphate, the Group IIIB metal monohydrogen phosphate, the Group IIIB metal dihydrogen phosphate, or mixtures in varying proportions of the Group IIIB metal monohydrogen and dihydrogen phosphates and/or mixtures in varying proportions of any of the above Group IIIB metal monohydrogen and dihydrogen phosphates with the Group IIIB metal phosphate. Such variations in catalyst composition can result from dependence of the catalyst composition on preparation conditions, such as temperature, concentration of reagents, stoichiometry of reagents, rate and order of reagent additions, pH of preparation, duration of preparation, volume and pH of water wash, duration of catalyst washing, and duration and temperature of catalyst drying. In any event, the Group IIIB metal acid phosphates obtained according to the general preparations referred to above are catalytically active for the production of, for example, polyalkylene polyamines.

Published European Patent Application 0115138 (the pertinent parts of which are incorporated herein by reference) discloses methods for preparing the catalysts comprising a phosphorous bonded to a Group IVB metal oxide support. Any appropriate liquid or liquifiable phosphorus compound can be used as a source of the phosphorus. For convenience, phosphoric acid will normally be used. However, other phosphorus compounds such as phosphoryl chloride ($POCl_3$), phosphorus acid, polyphosphoric acid, phosphorus halides, such as phosphorus bromide, alkyl phosphates and alkyl phosphites such as trimethyl phosphate, triethyl phosphate, trimethyl phosphite, triethyl phosphite, etc. may be utilized. Also, a diaminohydrogen phosphate such as diammonium hydrogen phosphate, $(NH_4)_2HPO_4$, dimethyldiamino hydrogen phosphate, diethylaminohydrogen phosphate, etc. may be used. The catalyst compositions are prepared by depositing a phosphorus compound on a support comprising an oxide of a group IVb transition metal oxide. The group IVb transition metal oxides include the oxides of titanium, zirconium, hafnium and thorium. Pellets of the group IVb metal oxide may be prepared by extrusion or by compaction in conventional pelleting apparatus using a pelleting aid such as graphite. The phosphorus compound can be on a powdered IVb metal oxide followed by pelleting and calcination. Preferably the catalyst composition is prepared by impregnating a preformed pellet. A suitable procedure to be used is to heat a liquid containing the liquid or liquefiable phosphorus compound at a temperature of about 100° to about 150°C. and to then add pellets in an amount about equal to the volume of the heated liquid. This treatment should be continued from about 0.5 to about 5 hours. At the end of that time, the resulting mixture of pellets and liquid is cooled, decanted to remove excess liquid followed by washing with an amount of water adequate to substantially completely remove unadsorbed liquid. Temperatures above 150°C. can be used, if desired, but there is no particular advantage in doing so. It will be understood that the phosphorous that is present on a thus-treated pellet is not present as elemental phosphorous, but rather as phosphorous that is chemically bound, probably as an oxide, to the group IVb metal oxide support. However, the exact nature of the bonding is not completely understood.

European Patent Application 0115138 discloses an amount of phosphorous that is bonded or otherwise adheres to the support is a function of heating and other conditions used in the treating step and is also a function of the chemical identity of the phosphorous compound that is used as a source of phosphorous. Under the treating conditions exemplified above, at least about 2.5 weight percent of phosphorous is caused to bond or otherwise permanently adhere to the pellets. There is an upper limit to the amount of phosphorous that bonds or otherwise permanently adheres to the support. This upper limit is, as indicated, a function of both the treating conditions and the chemical used as a source of the phosphorous. Normally, the maximum amount of phosphorous that can be caused to bond or otherwise permanently adhere to the pellets is within the range of about 5 to 10 weight percent. As a matter of convenience, the normal practice is to use only one chemical as the phosphorous source (e.g., phosphoric acid). However, mixtures of two or more such reagents may be used, if desired. Calcining is not mandatory if the pellets are impregnated at least at about 100°C., but the pellets can be calcined, if desired. Calcining is conducted for 2 to 24 hours at a temperature of 100°C. but below the temperature at which thermal destruction of the phosphorous bonding occurs. This can be determined experimentally for a particular catalyst. Temperatures above 900°C. should be avoided. A suitable calcining temperature range is normally 200° to 800°C. and, more preferably 500° to 700°C. Other procedures can be used in adding phosphorous to the group IVb metal oxide.

The pertinent parts of copending, commonly-assigned U.S. Application Serial No. 576,807, filed on February 7, 1984, are incorporated herein by reference. U.S. Ser. No. 576,807 discloses certain phosphorous acid or acid derivative compounds which are useful phosphorus-containing catalysts within the scope of the invention herein. The term phosphorus acid or acid derivative defines compounds having a P-X bond wherein P is a phosphorus atom bonded to a halogen, oxygen, sulfur or nitrogen atom and wherein X is a radical capable of (1) hydrolyzing to produce the corresponding phosphorus acid structure, or (2) exchanging with a hydroxyl group from the hydroxy alkylene reactant to provide a phosphorus ester.

The phosphorus acid or acid derivative catalyst of U.S. Ser. No. 576,807 is believed to function by forming with a alkanolamine or alkylene glycol compound a phosphorus ester in situ. For this reason, it is believed that a requirement for a good phosphorus catalyst is that it contain as a substructure an atom bonded to phosphorus that can be replaced readily by the oxygen atom of a hydroxyl group of the difunctional hydroxyl alkylene compound. Such a replaceable atom might be oxygen - (as in the case of phosphorus or phosphoric acid or their esters), halogen, nitrogen (as in the case of amides of phosphorus or phosphoric acids) or another atom that can be transformed into a phosphorus ester by a similar process.

Phosphorus-containing compounds such as trialkyl and triaryl phosphines and phosphine oxides, which contain no such exchangeable substructure, do not function as catalysts as defined in U.S. Ser. No. 576,807. Very sterically hindered phosphorus compounds such as hexaethyl phosphoric triamide, while containing the requisite exchangeable substructure and functioning to some extent, are less preferred catalysts because they undergo the exchange process with a alkanolamine or alkylene glycol hydroxyl moieties only slowly. Phosphorus acids are defined by those structures wherein X in the P-X radical is a hydroxyl radical. Acid derivatives are defined by structures wherein X is a substitute functional group. Various acid derivatives include: salts when -X is $-O^{-}M^{+}$ wherein $M^{+}$ is a mono or polyvalent cation; amides when -X is bonded to be phosphorus atom through a nitrogen atom; anhydrides when -X contains a second phosphorus atom bonded to the first phosphorus atom through an oxygen atom; esters when -X is -OR; and so on with regard to other functional groups defined by -X. The precise phosphorus acid or acid derivative structure is not critical so long as it fulfills the following two functional requirements - (when being used in the production of polyalkylene polyamines): (1) that it provides for the relatively selective production of predominantly linearly extended polyalkylene polyamines and (2) that it enables increased conversion rates for polyalkylene polyamine production when water is removed during the reaction, possibly due to the water-inhibited formation of a phosphorus intermediate compound during the reaction.

The phosphorus acids or acid derivative catalysts of U.S. Ser. No. 576,807 include those having the structure:

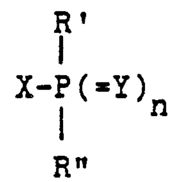

wherein Y is an oxygen or sulfur atom; n is 0 or 1; X is hydroxy, alkoxy, aryloxy, or the thio analogs of the foregoing, alkyl or aryl substituted amino, halo, or the salts or phosphorus anhydrides or thioanhydrides of the foregoing when X is hydroxy or mercapto; and R' and R" are hydrogen, alkyl, aryl or one of the groups previously defined by X.

Suitable phosphorus acid or acid derivatives of U.S. Serial No. 576,807 which can be employed included, for example, acidic metal or semi-metal phosphates, phosphoric acid compounds, and their anhydrides, phosphorus acid compounds and anhydrides, alkyl or aryl phosphates, alkyl or aryl phosphites, alkyl or aryl substituted phosphonic acids and phosphinic acids, alkali metal monosalts or phosphoric acid, phosphorus amides and phosphoric amides, the thio analogs of the foregoing, and mixtures of any of the above. Suitable acidic metal or semi-metal phosphates include boron phosphate, ferric phosphate, aluminum phosphate and the like. Suitable phosphoric acid compounds include aqueous or anhydrous phosphoric acids, such as orthophosphoric acid, pyrophosphoric acid, metaphosphoric acid, and condensed phosphoric acids such as polyphosphoric acids. Any commercially available mono-, di-, or trialkyl or aryl phosphate or phosphate ester can be employed. In addition, bis-(phosphates) and secondary phosphate esters, such as those disclosed in U.S. Patent No. 3,869,526 and U.S. Patent No. 3,869,527, respectively, can be utilized. Suitable alkyl or aryl substituted phosphonic acids or phosphinic acids include alkyl phosphonic acids, aryl phosphonic acids, alkyl phosphinic acids and aryl phosphinic acids. Examples of such phosphorus acid or acid derivative compounds include phenylphosphinic, ethylphosphonic, phenylphosphonic, naphthaphosphonic, and methylphosphinic acids; methyl phenylphosphonate, dimethyl phenylphosphonate, methyl phenylphosphinate, ethyl naphthaphosphinate, propyl methylphosphonate; and hexamethyl phosphoric triamide, hexaethyl phosphoric triamide and their analogous phosphorus triamides. Preferred phosphorus catalysts include hexamethyl phosphorus triamide, hexaethyl phosphorus triamide, boron phosphate, ferric phosphate, aluminum phosphate, phosphoric acid and phosphorus acid.

The amount of phosphorus acid or acid derivative catalyst of U.S. Ser. No. 576,807 utilized is a catalytically effective amount to cause condensation of the reactants to produce the desired organic amine condensation products. This quantity will vary depending upon the reaction conditions and catalyst utilized. Usually a catalytically effective amount will be from about 0.01 to about 10 mole percent and preferably from about 1 to about 3 mole percent, based on the moles of reactants used.

The pertinent parts of copending, commonly-assigned U.S. Patent Application Serial No. 606,000 filed on May 2, 1984, are incorporated herein by reference. U.S. Ser. No. 606,000 discloses certain phosphorus amide catalysts which are compounds having at least one phosphorus-nitrogen, i.e., P-N bond. Preferably, the P-N bond is part of a P-N-H or P-N-C substructure. Compounds containing suitable P-N bonds can have three, four, or five substituents about the phosphorus.

Suitable compound catalysts of U.S. Ser. No. 606,000 having three substituents about phosphorus can be defined by the formula:

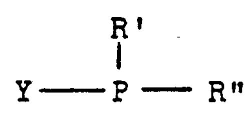

wherein Y is an unsubstituted or alkyl and/or aryl substituted amino radical; R' and R" are hydroxy, alkoxy, aryloxy, or their thioanalogs, hydrogen, alkyl, aryl, halo, or one of the groups previously defined by Y, and can be joined together with each other or with Y to form a phosphorus-containing heterocyclic ring. If R', R", or Y contains hydrogen bonded to O, S, or N, such as when R' or R" is hydroxy or mercapto or Y is monoalkylamino, then corresponding metal salts containing P-O-M, P-S-M, or P-N-M linkages, where M is a monovalent or polyvalent metal or semi-metal ion, and anhydrides, thioanhydrides, and condensed phosphorus amides containing respectively P-O-P, P-S-P, and P-N-P linkages can be suitable catalysts as well.

Suitable phosphorus amide catalysts of U.S. Ser. No. 606,000 having four substituents about phosphorus include those having the formula:

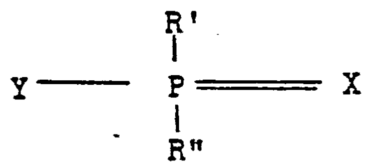

wherein X is an oxygen or sulfur atom, preferably oxygen, and Y, R', and R" are as defined above. As previously stated, corresponding metal and semi-metal salts and condensed phosphorus compounds may also be suitable.

Suitable phosphorus amide catalysts of U.S. Ser. No. 606,000 having five substituents about phosphorus include those having the formula:

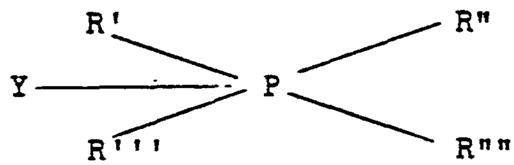

wherein Y is defined as above and R', R", R''', and R"" are as defined for R' and R" above. As previously stated, corresponding metal and semi-metal salts and condensed phosphorus compounds may also be suitable.

Suitable phosphorus amide compounds which can be employed include, for example, the following compounds or their alkyl or aryl derivatives: phosphoramidous acid, $H_2N\text{-}P(OH)_2$;

phosphordiamidous acid, $(H_2N)_2POH$;

phosphordiamidic acid, $(H_2N)_2P(O)(OH)$;

phosphoramidic acid, $H_2NP(O)(OH)_2$;

alkyl and aryl phosphonamidic acids, $RP(O)(OH)NH_2$;

alkyl and aryl phosphonamidous acids, $RP(OH)NH_2$;

esters and half-esters of the foregoing, e.g. $H_2NP(OEt)_2$;

metal salts of the foregoing, e.g. $H_2NP(O)_2K_2$;

triaminophosphine, $(H_2N)_3P$;

triaminophosphine oxide $(H_2N)_3P(O)$;

alkyl and aryl phosphonic diamides, $RP(O)(NH_2)_2$;

alkyl and aryl phosphonous diamides, $RP(NH_2)_2$;

alkyl and aryl phosphinous amides, $R_2P(NH_2)$;

alkyl and aryl phosphinic amides, $R_2P(O)(NH_2)$;

analogs of the foregoing substituted with alkyl or aryl groups on nitrogen, e.g. $R_2NP(OH)_2$;

and thioanalogs of the foregoing, e.g. $R_2NP(S)(OEt)_2$.

The alkyl or aryl substituents on these substances can be linked to phosphorus through more than one atom, so as to form cyclic members of the above classes containing such heterocyclic rings as:

1,3,2-diazaphospholidine,

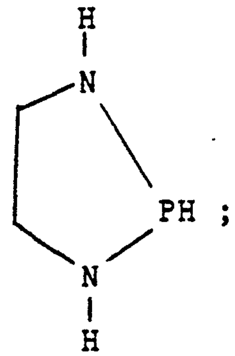

1,3,2-oxazaphospholidine,

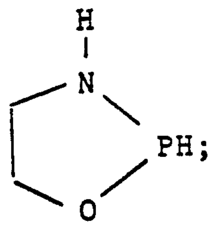

tetrahydro-2H-1,3,2-oxazaphosphorine,

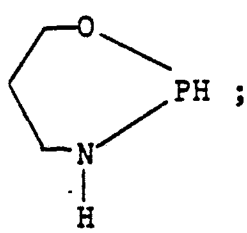

and the like. Such cyclic phosphorus amides can also be used as catalysts in the invention.

An additional class of phosphorus amides of U.S. Ser. No. 606,000 that can be useful as catalysts in the invention comprise azophosphoranes in which nitrogen is bound directly to phosphorus. Examples of such compounds include: 1,6-dioxa-4,9-diaza-5-phospha-(5-$P^V$)spiro [4.4]-nonane,

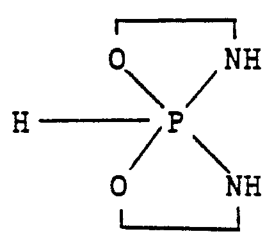

; and 2,3,5,6,8,8-hexahydro-8-methyl-[1,3,2]-oxazaphospholo-[2,3-b][1,3,2]oxazaphosphole,

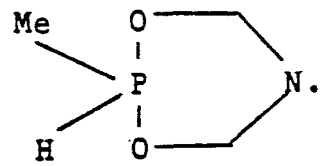

Preferred phosphorus amide catalysts of U.S. Ser. No. 606,000 include hexamethyl phosphorus triamide, hexaethyl phosphorus triamide and the phosphorus amide reaction product of ethylenediamine with phosphoric or phosphorus acid.

U.S. Patent No. 4,503,253 (the pertinent parts of which are incorporated herein by reference) discloses catalysts which are phosphorus or phosphoric acid on an inert support. Inert supports which can be utilized include silica, alumina, carbon silica-alumina clays and molecular sieves, e.g., aluminosilicates. The weight of phosphoric acid to support is variable and its optimum level will depend upon the reactants and operating conditions. Typically, the weight of phosphoric acid to support is from 1 to 85 percent.

Phosphorus-containing cation exchange resins can be used in this invention and can be prepared by the methods disclosed in U.S. Patent No. 4,324,917. The cation exchange resins provide exchangeable phosphorus-containing ions such as phosphonous, phosphonic, phosphoric and phosphorus. Preferably the resins useful here are weak-acid cation exchange resins containing one or more of the above phosphorus-containing exchangeable ions. Duolite ® resins available from Diamind Shamrock Corp. are typical commercial phosphorus-containing resins. Examples of these are Duolite ® ES-62, Duolite ® ES-63, and Duloite ® ES-65 which are the phosphonous, phosphonic and phosphoric acid types, respectively.

When a phosphorus-containing catalyst is used, the reaction temperature is preferably 220°C. to 350°C. (most preferably 260° to 300°C.) and most preferably the reaction pressure is 200 to 2,000 p.s.i.g.

Any of the non-resin phosphorus-containing catalysts useful in the invention can be supported on carriers, such as, silica, silica-alumina, silica-titania, alumina, diatomaceous earth (Kieselguhr) and any other conventionally-employed inert reactor packing material. Generally, the catalysts are supported. The active catalyst species are provided on the surface of the support through, for example, coating or impregnation. The catalyst (say metal) components on the support often comprises about 1 to 50, say, about 3 to 30, weight percent of the catalyst. Useful supports can be porous and have surface areas of from about 0.1 to 500, say, about 0.3 to 100, square meters per gram.

The catalyst can be of any convenient size or shape. Catalysts can be made in the form of powders, spherical or conical pellets, extruded strips and the like. Impregnated spherical pellets ranging in diameter from 1/8 inch to 3/16 inch and extruded strips of a cylindrical-type shape ranging from 1/32 inch to 1/2 inch in length are typical of those which can be used as supports. Often, for commerical-scale operations, the pellets range in diameter from about 0.1 to 1 centimeter.

By separation zone is meant those apparatus, e.g., stills, distillation columns, condensors, adsorbers, flash off units or evaporators, in which a reaction product stream is separated into at least one component.

The separation zone can be a conventional separation zone used for the separation of any reaction product stream containing at least one organic amine condensation product. For example, an organic amine condensation product is separated from the first reaction product stream in the separation zone and then a different organic amine condensation product is separated from the second reaction product stream in the same separation zone.

U.S. Patent No. 4,00,539 (the pertinent parts of which are incorporated herein by reference) discloses a separation scheme which could be used in the invention process.

Initially, for example, the reaction product stream exiting from the reactor can be passed into a conventional flashing unit to flash off the diluent and/or excess ammonia, (plus any hydrogen). This allows more efficient operation of the distillation columns.

After any diluent and/or excess ammonia (plus any hydrogen) have been flashed off, for example, the reaction product stream can be fed into a series of distillation columns to remove the various reaction products and by-products and unreacted feed components.

PREPARATION OF POLALKYLENE POLYAMINES

Polyalkylene polyamines can be prepared by reacting an alkylenediamine having at least two amino groups (e.g., ethylenediamine) and an alkanolamine having at least one amino group (e.g., ethanolamine). Noncyclic polyalkylene polyamines, such as, diethylenetriamine and cyclic polyalkylene polyamines, such as piperazine, can be prepared.

For the production of polyalkylene polyamines, the reactor is operated most preferably at a pressure of 200 to 2,000 p.s.i.g.; most preferably at a temperature of 260° to 300°C., when a phosphorus-containing catalyst is used or preferably at a temperature of 150° to 235°C. when a reductive amination catalyst is used. Also, preferably a metal acid phosphate catalyst or Ni reductive amination catalyst is used in the reactor. Any amination catalyst can be used. Preferably the reaction is conducted in the vapor phase or supercritical phase.

The alkyleneamine having at least two amino groups is preferably an unbranched alkylene moiety, such as, ethylenediamine, and preferably has primary amino groups. The alkanolamine preferably has a primary or secondary hydroxyl moiety and preferably amino group(s). Preferably, the alkanolamine has an unbranched alkylene moiety.

The alkanolamine compounds used in the invention process include those represented by the formula:

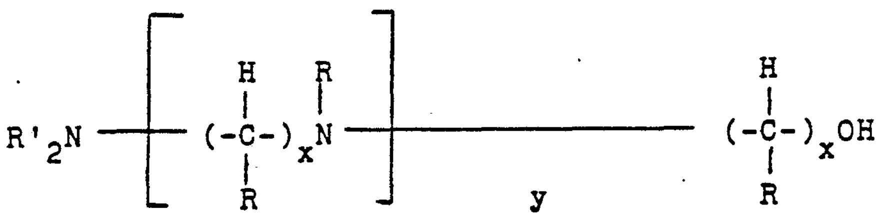

wherein R is hydrogen or a lower alkyl ($C_1$ to $C_4$) radical, R' is hydrogen or an alkyl ($C_1$ to $C_{25}$) radical, x is a number from 2 to 6, and y is a number from 0 to 3. Examples of suitable alkyl radicals are the lower ($C_1$ to $C_4$) alkyls, such as, methyl, ethyl and butyl, and higher alkyls, such as, octyl, decyl and octadecyl. Methyl is the preferred lower alkyl radical. However, it is preferred that R and R' both are hydrogen; thus the alkanolamine would contain a primary amino group. Examples of useful alkanolamine compounds are the ethanolamines, isomeric propanolamines, N-(2-aminoethyl) ethanolamine, N-methylethanolamine, N,N-dimethylethanolamine, N,N,N'-trimethylaminoethylethanolamine and the like.

The alkyleneamine reactants used in the invention process are represented by the formula:

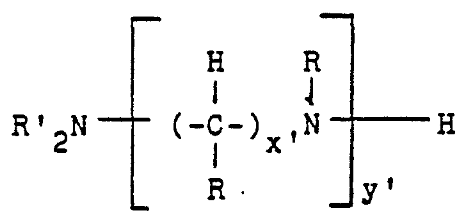

wherein R is hydrogen or a lower alkyl ($C_1$ to $C_4$) radical, R' is hydrogen or an alkyl ($C_1$ to $C_{25}$) radical, x' is a number from 2 to 6, and y' is a number from 1 to 4. Examples of suitable alkyl radicals are the lower ($C_1$ to $C_4$) alkyls, such as, methyl, ethyl and butyl, and higher alkyls, such as, octyl, decyl and octadecyl. It is preferred that R and R' are both hydrogen. The preferred lower alkyl radical is methyl. Examples of useful alkyleneamine compounds are 1,3-propylenediamine, N-methylpropylenediamine, 1,2-propylenediamine, diethylenetriamine, tributylenetetramine, triethylenetetraamine, N,N,N'-trimethyldiethylenetriamine, noncyclic isomers of triethylenetetramine, noncyclic isomers of tetraethylenepantamine, N-methylethylenediamine, N,N-dimethylethylenediamine and ethylenediamine, which is the preferred alkyleneamine compound.

In the reactor, the temperature for the reaction depends upon the particular starting material, ratios of reactants, and most importantly, the activity of the catalyst used. Generally, in this embodiment of the process of the invention, temperatures within the range of about 125° to 425°C. are suitable. A diluent gas can be utilized.

A relatively high pressure of the reaction is also preferred. Normally, a diluent, such as, hydrogen, helium, water, nitrogen, methane or argon, can be added to increase the pressure in a batch reactor and the volumetric flow in a fixed bed reactor. The pressure at the time of reaction should normally be within the range from about 50 to about 4,000 p.s.i.g. In any event, the pressure should be selected such that the reaction feedstream is maintained in the desired phase.

Ammonia or a primary amine or a secondary amine can also be used in the feed to the reactor. In fact, the reaction preferably is conducted with ammonia present in the feed. The ammonia is both a reactant and a diluent (when excess of it is present) in the invention process. When ammonia is used, the mole ratio of ammonia (or the primary amine or the secondary amine) to the combination of ethylenediamine and monoethanolamine is usually in the range of 20:1 to 1:20 and preferably in the range of 12:1.25 to 1:1. Ammonia and the preferred primary and secondary amines which are used in the invention process are represented by the formula:

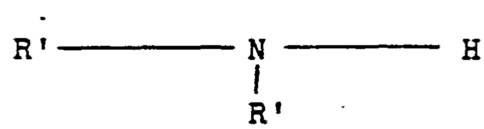

wherein R' is hydrogen or an alkyl ($C_1$ to $C_{25}$) radical, preferably a lower alkyl ($C_1$ to $C_4$) radical, such as methyl or ethyl. Stoichiometrically, one molecular unit of ammonia or amine (primary or secondary) is required per molecular unit of hydroxyl group. However, the formation of diethylenetriamine is favored by the presence of excess ammonia, but a practical limit on the amount of ammonia employed exists due to energy consumption. Useful amine feedstocks include monomethylamine, dimethylamine, monoethylamine, diethylamine, octylamine and octadecylamine.

Use of secondary amines instead of ammonia leads to polyalkylene polyamines containing terminal dialkylamino groups. Alternatively, use of primary amines instead of ammonia leads to polyamines which contain randomly distributed monoalkylamino groups.

The invention process generally predominantly produces noncyclic polyalkylene polyamines if the proper procedures, conditions, etc., are followed and observed. Noncyclic polyalkylene polyamines that are generally produced by the reaction of alkanolamine, plus an alkylenediamine are represented by the formula:

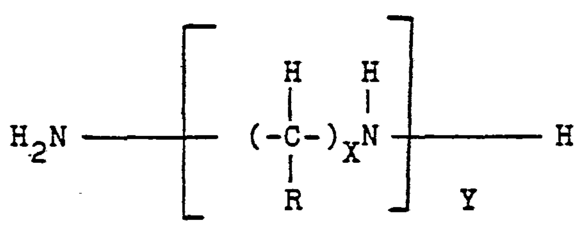

wherein R is hydrogen or a lower alkyl ($C_1$ to $C_4$) radical, preferably a methyl radical, X is a number from 2 to 6, Y is a number from 2 to 7, and X can vary for a given value of Y. Examples of such noncyclic polyalkylene polyamines that are produced are dipropylenetriamine, tributylenetetramine, di(2-methylethylene) triamine, tri(2-methylethylene)tetramine, N-(2-amino-ethyl)-1,3-propylene, diethylenetriamine and the noncyclic isomers of tetraethylenepentamine. It is generally desirable not to coproduce significant amounts of by-products, particularly cyclic amines, especially piperazine and aminoethylpiperazine, that are of less commercial value than diethylenetriamine.

Cyclic polyalkylene polyamines that are produced by the reaction of (a) an alkyleneamine and an alkanolamine or (b) ammonia, an alkyleneamine and an alkanolamine or (c) an alkanolamine and ammonia are represented, for example by the following formula:

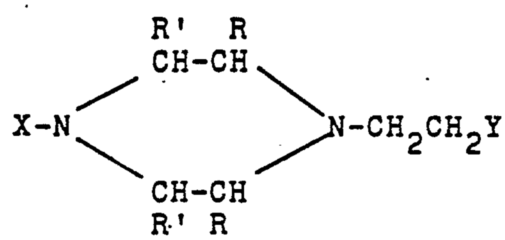

wherein R is hydrogen, an alkyl group containing 1 to 12 carbon atoms or a cycloalkyl grup containing 6 to 12 carbon atoms, R' is hydrogen or an alkyl group containing 1 to 4 carbon atoms, X is hydrogen or $-CH_2CH_2Y$ and Y is -OH or $-NH_2$.

Examples of cyclic polyalkylene polyamines are pierazine, N-(2-hydroxyethyl)piperazine, N,N-di(hydroxyethyl)piperazine, N-(hydroxyethyl)-2-methylpiperazine, N,N,'-di(hydroxyethyl-2-methylpiperazine, N-(hydroxyethyl)-2-ethylpiperazine, N,N'-di(hydroxyethyl)-2-ethylpiperazine, N-(hydroxyethyl)-2-butylpiperazine, N,N-di-(hydroxyethyl)-2-butylpiperazine, N-(hydroxyethyl)-2-dodecylpiperazine, N-(hydroxyethyl)-2-cyclohexylpiperazine, N-(hydroxyethyl)-2-hexylcyclohexylpiperazine, N,N'-(dihydroxyethyl)-2,5-dimethylpiperazine, N-(hydroxyethyl)-2,3,5,6-tetramethylpiperazine, N,N'-di(hydroxyethyl)-2,5-dimethylpiperazine, N-(hydroxyethyl)-2,5-diethylpiperazine, N-(hydroxyethyl)diethylenetriamine, N-(hydroxypropyl)diethylenetriamine, N-(2-hydroxy butyl)diethylenetriamine, N-(hydroxyethyl)-dipropylenetriamine, N-(hydroxypropyl)-dipropylenetriamine, N-(2-hydroxybutyl)-dipropylenetriamine and morpholine.

Predominantly noncyclic polyalkylene polyamines means greater than about 50 weight percent of linear and branched polyalkylene polyamines in the total polyamine product.

Noncyclic polyalkylene polyamines include polyalkylene polyamines having straight-chain and branched alkylene groups.

Polyethylene polyamines are useful as corrosion inhibitors, fabric softeners, lubricating oil additives, comonomers for polyamide resins, fungicides, surfactants, curing agents for epoxy resins and chelating agents. Diethylenetriamine is specifically useful as a solvent for sulfur, acid gases, various resins and dyes and as a saponification agent for acidic materials.

## PREPARATION OF TRIETHYLENEDIAMINE

Triethylenediamine or its carbon-substituted derivatives can be prepared by the invention process. Triethylenediamine is also termed diazabicyclo-(2.2.2)-octane or DABCO®. For example, triethylenediamine is prepared from N-aminoethylpiperazine. In the reaction mixture, the principal organic components are compounds in which the ethylene group ($-CH_2CH_2-$) has a nitrogen at one end and either a nitrogen or an oxygen at the other end.

For the production of triethylenediamine, the reactor is operated preferably at a pressure of about one atmosphere absolute and preferably at a temperature of 230° to 370°C. (most preferably 300° to 360°C.). Also, preferably a metal acid phosphate catalyst is used in the reactor and preferably the reaction is conducted in the vapor phase.

In order to produce diazabicyclo-(2.2.2)-octane and C-substituted diazabicyclo-(2.2.2)-octanes in the invention process, a compound having the formula:

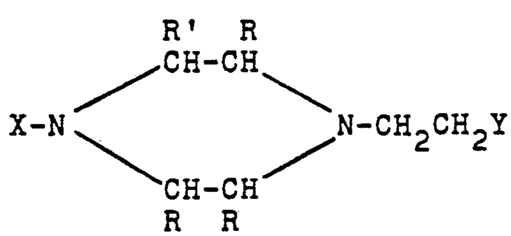

or a compound having the formula:

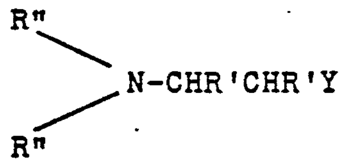

wherein R is hydrogen, an alkyl group containing 1 to 12 carbon atoms or a cycloalkyl group containing 6 to 12 carbon atoms, R' is hydrogen or an alkyl group containing 1 to 4 carbon atoms, R" is hydrogen or -CHR'CHR'Y, X is hydrogen or -$CH_2CH_2Y$ and Y is -OH or -$NH_2$, is contacted with the phosphorus-containing catalyst. (A reductive amination catalyst is not used to produce DABCO®.)

Examples of reactants within the first formula above are N-aminoethylpiperazine, N-hydroxyethylpiperazine, N,N'-di(aminoethyl)piperazine, N,N'-di-(hydroxyethyl)piperazine, N-(hydroxyethyl)-2-methylpiperazine, N,N,'-di(hydroxyethyl-2-methylpiperazine, N-(aminoethyl)-2-methylpiperazine, N,N'-di(aminoethyl)-2-methylpiperazine, N-(hydroxyethyl)-2-ethylpiperazine, N,N'-di-(hydroxyethyl)-2-ethylpiperazine, N-(aminoethyl)-2-dodecylpiperazine, N-(aminoethyl)-2-ethylpiperazine, N-(hydroxyethyl)-2-butylpiperazine, N,N-di(hydroxyethyl)-2-butylpiperazine, N-(hydroxyethyl)-2-dodecylpiperazine, N-(aminoethyl)-2-doedecylpiperazine, N-(hydroxyethyl)-2-cyclohexylpiperazine, N-(hydroxyethyl)-2-hexylcyclohexylpiperazine, N-(hydroxyethyl)-2,5-dimethylpiperazine, N,N'-di-(hydroxyethyl)-2,5-di-methylpiperazine, N-(hydroxyethyl)-2,3,5,6-tetramethyl-piperazine, N-(aminoethyl)-2,5-diethylpiperazine, N,N'-di-(hydroxyethyl)-2,5-dimethylpiperazine, N-(hydroxy ethyl)-2,5-diethylpiperazine and mixtures thereof. When hydroxyethylpiperazine or other as such hydroxy compounds is used as the starting compound, water is a by-product.

Examples of reactants within the second formula above are monoethanolamine, diethanolamine, triethanolamine, monoisopropanolamine, diisopropanolamine, triisopropanolamine, sec.-butanolamine, di-sec.-butanolamine, di-sec.-hexanolamine, sec.-hexanolamine, aminoethylethanolamine, aminopropyl ethanolamine, aminopropyl propanolamine, N-(hydroxyethyl)diethlenetriamine, N-(hydroxypropyl)-diethylenetriamine, N-2-hyroxybutyl) diethylenetriamine, N-(hydroxyethyl)-dipropylenetriamine, N-(hydroxypropyl)-dipropylenetriamine, N-(2-hydroxybutyl) dipropylenetriamine, ethylenediamine, diethylenetriamine, 1,2-propylenediamine, di-1,2-propylenetriamine, and mixtures thereof.

Although the feed materials to be employed can be substantially anhydrous, aqueous feed stocks containing up to about 50 weight percent of water can be used.

The cyclization reaction can be conducted in the absence of ammonia, but preferably the reaction is conducted in the presence of from about 1 to about 15 moles of ammonia per mole of substituted piperazine feed stock. More preferably, from 3 to about 10 moles of ammonia are employed.

Hydrogen can also be employed in the process, for example, as a purge or carrier gas.

Although the reaction is preferably conducted at atmospheric pressure, subatmospheric or superatmospheric pressures can be employed.

The reaction temperature to be employed is often within the range of 230° to 370°C., and is most preferably within the range of 300° to 360°C.

When the feed stock is a compound falling within the second formula above, from 1 to 15 moles of ammonia should be employed per mole of feed stock.

U.S. Patent Nos. 3,297,701 and 3,080,371 (the pertinent parts of such patents are incorporated herein by reference) discloses the conditions generally used in preparing diazabicyclo-(2.2.2)-octane and its derivatives from the above-mentioned starting materials.

The effluent from the reaction zone contains significant amounts of compounds in which the ethylene group (-$CH_2$-$CH_2$-) has a nitrogen at one end and either a nitrogen or an oxygen at the other end. Moreover, such effluent contains at least minor amounts of the compounds present in the feedstock. Representative members of the compounds which might be formed as conversion products from the reaction zone include ethylenediamine, diethylenetriamine, triethylenediamine, piperazine, diethanolpiperazine, di(betaaminoethyl) piperazine, and triethylenetetramine. The thermal decomposition products from such compounds are also present on the effluent from the reaction zone. Representative members of such thermal decomposition products include pyrazine and lower alkyl pyrazines of the formula $C_4R_nH_{4-n}N_2$ in which R is selected from the group consisting of methyl and ethyl groups. The organic effluent from the zone for the synthesis of triethylenediamine over a catalyst consists almost entirely of a mixture of the thermal decomposition products derived from and the compounds in which the ethylene group has a nitrogen at one end and either a nitrogen or an oxygen at the other end. Substantially all of the organic components (cyclic, polycyclic, linear or complex) of the effluent can be conveniently classified as members of the class $R_2NCR_nCR_nQR_n$ in which Q is selected from the group consisting of nitrogen and oxygen and in which n is 1 or 2 and in which the R's are the same or different and represent hydrogen or an organo group. Most of the organo groups either have not more than 2 carbon atoms or are portions of cyclic or linear groups in which nitrogen atoms are so positioned that the carbon chains contain not more than 2 atoms.

TEDA is particularly useful as an accelerator or catalyst in urethane systems empolying a variety of isocyanates and polyols as reactants.

## PREPARATION OF ETHYLENEDIAMINE

Ethylenediamine can be prepared from ammonia and monoalkanolamine and can be prepared from a stream containing ammonia, monoethanolamine, diethanolamine and triethanolamine, as produced by the direct reaction of ethylene oxide and ammonia.

For the production of ehtylenediamine, the reactor is operated normally at a pressure of 50 to 4,000 p.s.i.g., preferably a pressure of greater than 100 p.s.i.g., and most preferably at a pressure of 200 to 2,000 p.s.i.g. When a reductive amination catalyst is used, preferably the reaction temperature is 150° to 235°C. When a phosphorus-containing catalyst is used, preferably the reaction temperature is 220° to 350°C. Preferably the reaction is conducted in the vapor phase or supercritical phase.

The pertinent parts of U.S. Patent No. 4,400,539 are incorporated herein by reference. Alkanolamines are prepared by the conventional reaction of ammonia and ethylene oxide. Any of the processes described in the prior art which involve the reaction of ethylene oxide with ammonia to produce a mixture of monoethanolamine, diethanolamine and triethanolamine can be used. A desirable process is one which produces a mixture in which monoethanolamine is present in amounts greater than 50 weight percent of the total concentration of alkanolamines. Illustrative of such processes are those described in U.S. Patent Nos. 2,196,554, 3,697,598 and 3,723,530.

The reaction converts the products of the reaction of ethylene oxide and ammonia, which products contain, inter alia, monoethanolamine, diethanolamine and triethanolamine, to ethyleneamines. In the typical practice of the invention, the reaction serves to convert such products into ethylenediamine. As mentioned above, the reaction of ethylene oxide and ammonia produces a stream which contains essentially ammonia, monoethanolamine, diethanolamine and triethanolamine. The amount of ammonia in the product mixture is subject to the amount of ammonia which is utilized in the reaction with ethylene oxide. In the typical case the amount of ammonia which will be used will be vastly in excess of the stoichiometry of the reaction to produce the product mixture and therefore the available ammonia which is used in the reaction between ethylene oxide and ammonia will in large part be adequate from the subsequent amination reaction to produce the ethyleneamines. The feed stream can also contains recycled monoalkanolamine and optionally ammonia. Usually the feed stream contains at least 70 weight percent monoethanolamine based on the total ethanolamines content, not more than about 30 weight percent diethanolamine, same basis, not more than 15 weight percent of triethanolamine determined on the same basis, and the sum of the diethanolamine and triethanolamine does not exceed 30 weight percent of the total ethanolamine content thereof. The amination feed stream also contains ammonia in an amount which is in stoichiometric excess of the alcoholic hydroxyl groups which are present in the amination feed stream. Usually there are contained at least 10 moles of ammonia for each mole of ethanolamine present in the feed stream. The amination feed stream can also possess a limited amount of water. The water content in the feed stream can range between 0 weight percent to 10 weight percent, based on the weight of the amination feed stream.

Gaseous diluents such as nitrogen, water, helium, methane and the like can also be present in the reaction zone. Such gaseous diluents can be utilized.

The reaction temperature is broadly about 125° to 425°C. Preferably the feed stream within the reaction zone is under supercritical fluid or vapor phase conditions (although the liquid phase can be used). Therefore, the pressure within the reaction zone should preferably be correlated with

the temperature so as to achieve either the supercritical fluid conditions or a vapor phase condition. The reaction pressure broadly ranges between 50 and 4,000 p.s.i.g.

After the reaction product stream is removed from the reaction zone, it is subjected to a variety of separation steps from the purpose of removing the various components contained therein. For example, the effluent gas stream from the amination zone will be subjected to distillation to remove water, ammonia ethylenediamine, monoethylpiperazine (which can be recycled), hydroxyethylpiperazine, aminoethylethanolamine, tetraethylenepentamine, diethylenetriamine, aminoethylpiperazine, piperazine, triethylenetetramine, diethanolamine and triethanolamine. Because of the vast differences between the boiling points of monoethanolamine, diethanolamine and triethanolamine, the separation of monoethanolamine from the composition is very readily obtained and an extremely pure stream of monoethanolamine can be produced by simple distillation. In the normal course, the monoethanolamine which can be obtained by distillation will contain at least 99 weight percent of monoethanolamine with extremely small amounts of piperazine, diethylenetriamine, aminoethylpiperazine, hydroxyethylpiperazine, and the like, being present.

PREPRARATION OF ALKYLAMINES

Aliphatic amines, such as, alkyl amines, can be prepared by reacting an aliphatic alcohol, such as, an alkanol, and an aliphatic aldehyde, with ammonia or a primary amine or a secondary amine.

For the production of the methylamines, the reactor is operated most preferably at a pressure of 200 to 2,000 p.s.i.g. and most preferably at a temperature of 325° to 375°C. For the production of higher alkylamines, the reactor is operated most preferably at a pressure of 100 to 2,000 p.s.i.g. and preferably at a temperature of 150° to 200°C. A metal acid phosphate catalyst is preferably used for the production of methylamines; and a reductive amination catalyst (e.g., Ni) is preferably used for the production of higher alkylamines. Preferably the reaction is conducted in the vapor phase or supercritical phase.

The alkanols have the formula:

$R_1CH_2OH$

wherein $R_1$ is a straight chain or branched chain alkyl group having 1 to 10 carbon atoms. Examples are methanol, ethanol, propanol, isobutanol and octanol.

The alkyl aldehydes have the formula:

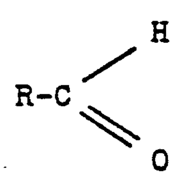

wherein R is a straight chain or branched chain alkyl group having 1 to 10 carbon atoms. Examples are formaldehyde, acetaldehyde, prionaldehyde, isobutyraldehyde and n-valeraldehyde.

The primary or secondary alkyl amines having the formula:

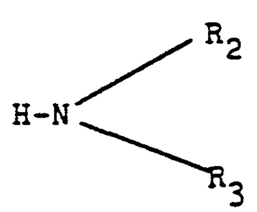

wherein $R_2$ and $R_3$ each is a hydrogen atom or a straight chain or branched chain alkyl group having 1 to 24 carbon atoms. Examples of such amines are dimethyl amine, diethylamine, methylamine and heptylamine.

The product is an alkyl amine having the formula:

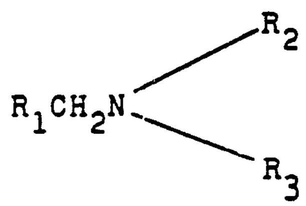

wherein $R_1$, $R_2$ and $R_3$ have the meanings set out just above.

Of particular interest is the catalyzed reaction of butanol and ammonia to produce monobutylamine, dibutylamine and tributylamines.

Generally, the mole ratio of ammonia to butanol can range from about 1:1 to 5:1, and preferably ranges from about 1.5:1 to 2.5:1. If butylamine is the desired product, it is advantageous to carry out the process with the proportion of ammonia to butanol in a stoichiometric excess, e.g. up to about 3:1 or more.

In the preparation of the methylamines, the reaction is broadly 230° to 430°C. If the temperature is too low, the conversion of methanol and ammonia to methylamines will be low. If the temperature is too high, hydrocarbon by-product formation and catalyst coking becomes a significant problem.

The pressure utilized for carrying out the reaction is broadly about 1 to 75 atmospheres.

PREPARATION OF MORPHOLINE AND ITS DERIVATIVES

Morpholine and morpholine compounds can be prepared from diglycolamine and diglycolamine compounds, respectively.

For the production of morpholine and its derivatives, the reactor is normally operated at a pressure of 50 to 4,000 p.s.i.g., and most preferably a reaction temperature of 220° to 350°C. is used. Also, preferably a metal acid phosphate catalyst or a Ni catalyst is used in the reaction. Preferably the reactor is conducted in the vapor phase or supercritical phase.

Morpholine compounds having the formula:

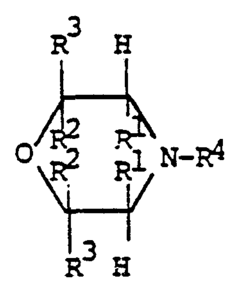

wherein $R^1$, $R^2$ and $R^3$, which may be the same or different, each is hydrogen or an alkyl radical, and $R^4$ is hydrogen or a substituted or unsubstituted alkyl, cycloalkyl, saturated heterocyclic or aryl radical, can be prepared by contacting a glycol having the formula:

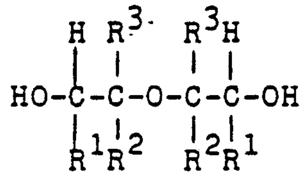

wherein $R^1$, $R^2$ and $R^3$ have the meanings given above, with ammonia or an amine having the formula:

$H_2N-R^4$

wherein $R^4$ has the meaning given above, in the presence of a phosphorus compound, at a temperature of from 200 to 350°C., under a pressure sufficient to maintain the reactants in the liquid phase.

By varying the glycol and the ammonia or amine utilized, one can achieve, for example, C-(alkyl substituted) morpholines or N-(substituted) morpholines. The primary amine may be a substituted or unsubstituted aryl amine or a substituted or unsubstituted alkyl amine.

The N-(substituted) morpholine compounds that can be produced have the formula above in which $R^4$ is an alkyl radical, a substituted alkyl radical, an aryl radical or a substituted aryl radical. Examples of these compounds are N-methyl morpholine, N-phenyl morpholine, N-(aminoethyl) morpholine, N-(2-N',N'-dimethylaminoethyl) morpholine and N-methyl-2,6-di-ethyl morpholine.

$R^1$, $R^2$, $R^3$ and $R^4$ may contain substituted moieties which are nondeleterious to the reaction, for example oxy, thio or tertiary amino moieties.

The glycol that can be used has the general formula above wherein $R^1$, $R^2$ and $R^3$ are independently an alkyl radical (e.g., having 1 to 4 carbon atoms) or hydrogen. The preferred glycol is diethylene glycol.

The primary amine that can be utilized has the formula above wherein $R^4$ has the meaning given above. Examples of suitable primary amines are methylamine, ethylamine, aniline, 2,6-dimethylaniline, the naphthylamines, N-(2-aminoethyl)morpholine, N-(2-aminoethyl) piperazine, and C-(aminoalkyl)morpholines.

Additionally, $R^4$ may contain substituted moieties which are nondeleterious to the reacton such as oxy, thio or tertiary amino moieties. The only requirement of the above compounds is that they contain a nitrogen moiety having two labile hydrogens and that the constituents or moieties linked with the nitrogen are not, or do not contain, constituents deleterious to the reaction. For example, primary amines containing a second primary amine group (so that the primary amine can be an alkylene diamine) or a hydroxy group can be used, but they will often substantially increase the by-products and side reactions.

The diglycolamine compounds can be, for example, alkyl diglycolamines, wherein each of the alkyl groups contains 1 to 6 carbon atoms. In such case, the morpholine derivative products would be alkyl morpholines, wherein each of alkyl groups contains 1 to 6 carbon atoms.

The reaction is broadly conducted at a temperature between 250° and 350°C. Preferably reaction conditions are chosen which minimizes the production of dioxane by-product.

Normally, the glycol and ammonia are reacted at molar ratios of from 1:1 to 20:1, preferably 2:1 to 10:1, moles of ammonia per mole glycol compound. Normally, the glycol and the primary amine are reacted at molar ratios from 0.5:1 to 20:1, and preferably 1:1 to 10:1, moles of glycol per mole of primary amine.

Illustrative of a preferred embodiment of the invention is an interchangeable separation scheme for five different organic amine condensation product streams. The interchangeable separation scheme uses the five distillation column system shown in Figure 1. The particulars are shown in the following Table:

TABLE[1, 7]

| Product Separated | First[9] Distillation Column (20) | | Second[10] Distillation Column (30) | | Third[11] Distillation Column (40) | | Fourth[11] Distillation Column (50) | | Fifth[11] Distillation Column (60) | |
|---|---|---|---|---|---|---|---|---|---|---|
| EDA/DETA | $NH_3$ | 175°C. | $H_2O$ | 175°C. | EDA[3]/PIP[4] 117°C./146°C. | | MEA | 170°C. | DETA | 207°C. |
| Morpholine | $NH_3$ | 175°C. | $H_2O$ | 175°C. | Morpholine 128°C. | | DGA | 221°C. | DEG | 246°C. |
| TEDA | $NH_3$ | 175°C. | $H_2O$ | 175°C. | PIP/nonane[2] 133° - 136°C. | | DETA/1,4 BDO 180°C. | | HEP | 246°C. |
| Butylamines | $NH_3$ | 175°C. | n-butylamine 78°C. | | $H_2O$/butanol[6,8] azeotrope 93°C. | | n-dibutylamine[6] 160°C. | | n-tributylamine[6] 213°C. | |
| EDA | $NH_3$ | 175°C. | $H_2O$ | 175°C. | EDA | 117°C. | PIP | 140°C. | MEA | 170°C. |

Table (Continued)

Notes:

1. The pressure in each distillation column is atmospheric pressure unless otherwise noted.
2. Nonane is a hydrocarbon extractant.
3. EDA comes off overhead.

3. EDA comes off overhead.

4. PIP comes out the side of the column.

5. Conducted at 90 mmHg absolute.

6. A countercurrent water scrub is used in the distillation column.

7. The boiling points can be lowered lowering the pressure in the columns.

8. The $H_2O$/butanol azeotrope is taken off the top of column 40 to an azeotrope separation tank (decantor). The butanol layer is returned to the top of column 40; and water is removed at the base of the separation tank.

9. At 30 p.s.i.g.

10. At 1220 mmHg absolute.

11. The atmospheric pressure indicated is only for illustrative purposes. In practice a subatmospheric pressure would be used to minimize decomposition.

The following compound abbreviations apply herein:

EDA -ethylenediamine

MEA -monoethanolamine

PIP -piperazine

AEP -aminoethylpiperazine

DETA -diethylenetriamine

TETA(NC) -triethylenetetramine (noncyclic isomers)

TETA(C) -tetraethylenepentamine (cyclic isomers)

TEPA(NC) -tetraethylenepentamine (noncyclic isomers)

TEPA(C) -tetraethylenepentamine (cyclic isomers)

HVY(NC) -pentaethylenehexamine and higher oligomeric polyethylene amines

DGA -diethyleneglycolamine

DEG -diethyleneglycol

TEDA -triethylenediamine

AEEA -aminoethylethanolamine

HEP -hydroxyethylpiperazine

1,4-BDO -1,4-butanediol

The following example, based upon the equipment shown in the figure, illustrate the invention:

THE EXAMPLE

100 moles of diethylene glycol and 200 moles of ammonia are fed to reactor 10, which is operated at a temperature of 285°C. and 1,500 p.s.i.g Reactor 10 contains a lanthanum hydrogen phosphate catalyst impregnated on a porous alpha-alumina support. The liquid hourly space velocity of the gaseous feed is 3.5 $hr^{-1}$. The reaction is conducted in the vapor phase.

The ammonia is flashed off from reaction product effluent in flashing unit 52, which is operated at 85°C. and 360 p.s.i.g. (ranges: 50° to 150°C.; and 100 to 1,000 p.s.i.g.). The ammonia, after condensing, (54) is recycled to feed line 88. Ammonia stripping still 20, which further removes ammonia from the reaction product effluent, is operated at 175°C. and 30 p.s.i.g. (ranges: 100° to 200°C.; and atmospheric pressure to 100 p.s.i.g.). That ammonia is also recycled to reactor feed line 88. Water is removed from the reaction product effluent in distillation column 30, which is operated at 1,220 mmHg absolute and 180°C. (ranges: 150° to 250°C.; and 100 mmHg absolute to 30 p.s.i.g.). Then the morpholine product is removed via line 72 from the reaction product effluent in distillation column 40, which is operated at 135°C. and 100 mmHg absolute (ranges: 100° to 200°C.; and 50 mmHg absolute to atmospheric pressure). The bottoms thereof are fed into distillation column 50, wherein diglycolamine from the top of the column is recovered or recycled to feed line 88. Column 50 is operated at 50 mmHg absolute and 165°C. - (ranges: 120° to 200°C.; and 5 mmHg absolute to atmospheric pressure). Diethylene glycol is then removed from the reaction product effluent by means of distillation column 60, which is operated at 10 mmHg absolute and 160°C. (ranges: 1 to 100 mmHg absolute; and 120° to 200°C.). The diethylene glycol is recycled to the reactor feed. The remaining heavies are removed from the bottom of column 60.

After 48 hours, the production of morpholine is stopped and the entire system is purged and cleaned. Then 33 moles of ethylenediamine, 67 moles of monoethanolamine and 200 moles of ammonia are fed per hour to reactor 10, which is operated at a temperature of 300°C. and 1,100 p.s.i.g. Reactor 10 contains a lanthanum hydrogen phosphate catalyst impregnated on a porous alpha-alumina support. The gas hourly space velocity of the gaseous feed is 25 $hr^{-1}$. The reaction is conducted in the vapor phase.

The ammonia is flashed off from the reaction product effluent in flashing unit 50, which is operated at 85°C. and 360 p.s.i.g. (ranges: 50° to 150°C.; and 100 to 1,000 p.s.i.g.). The ammonia, after being condensed (54), is recycled to feed line 88. Ammonia stripping 20, which further removes ammonia from the reaction product effluent, is operated at 175°C. and 30 p.s.i.g. (ranges: 100° to 200°C.; and atmospheric pressure to 100 p.s.i.g.). That ammonia is also recycled to reactor feed line 88. Water is removed from the reaction product effluent in distillation column 30, which is operated at 1,220 mmHg absolute and 180°C. (ranges: 150° to 250°C.; and 100 mmHg absolute to 30 p.s.i.g.). - (The azeotrope of water and ethylenediamine in column 30 is broken by extractive distillation using unreacted MEA.) Ethylenediamine is removed via line 72 from the reaction product effluent in distillation column 40, which is operated at 145°C. and 140 mmHg absolute (ranges: 100° to 200°C.; and 50 mmHg absolute to atmospheric pressure). An azeotrope of water and ethylenediamine is recycled via line 46 to column 30. Optionally, the azeotrope 46 instead is recycled via lines 46, to feed line 88. Also ethylenediamine is removed from the top portion of the still and is recycled to feed line 88. The centre strip of column 40 is recycled to column 30. The bottoms thereof are fed into distillation column 50, wherein monoethanolamine from the top of the column is recycled to feed line 88. Column 50 is operated at 45 mmHg absolute and 145°C. - (ranges: 100° to 180°C.; and 10 mmHg absolute to atmospheric pressure). Diethylenetriamine is then removed from the reaction product effluent by means of distillation column 60, which is operated at 110 mmHg absolute and 175°C. (ranges: 20 mmHg absolute to atmospheric pressure and 150° to 250°C.). The remaining heavies containing AEP, AEEA, TETA and TEPA are removed from the bottom of column 60 and sent to a batch still run at conventional conditions to separate. such components as desired.

Copending, commonly-assigned patent application entitled "Interreactor Separator", (D-14871), concurrently filed with this application, is incorporated herein by reference. Such application discloses the use of a reductive amination reaction zone from which its effluent is separated into a, preferably, gaseous, MEA-and EDA-containing phase and a liquid, DETA-rich phase. The gas phase can be used for recycle (advantageously, it is at high pressure and suitable for recycle without undue energy penalties) or as a feed to another reactor which can use a reductive amination or other type (e.g., phosphorus-based catalyst) of catalyst.

Copending, commonly-assigned patent application entitled "Improving The Quality Of Cataclytically Prepared Polyalkylene Polyamines", (D-14872), concurrently filed with this application, is incorporated herein by reference. Such application discloses processes for making polyalkylene polyamines from ethanolamines and a nitrogen compound (e.g., ammonia or alkyleneamine) using a phosphorus-based catalyst in the presence of sufficient hydrogen to enhance color and reduce odor.

Copending, commonly-assigned patent application entitled "Conversion Of Oxygen-Containing Polyamines", (D-14873) concurrently filed with this application, is incorporated herein by reference. Such application discloses, in its broadest aspect, passing an oxygenated, polyethylenepolyamine feed in the presence of a nitrogen compound to a reaction zone containing a phosphorus-based catalyst. Advantageously, this feed is obtained from a polyethylenepolyamine reactor having a reductive amination catalyst. The feed typically contains AEEA.

Copending, commonly-assigned patent application entitled "Two Reactor Scheme Using Only Phosphorus-Containing Catalyst", (D-14874), concurrently filed with this application. Such application discloses passing an oxygenated, polyethylenepolyamine feed in the presence of nitrogen compound to a reaction zone containing a phosphorus-based catalyst. This feed is obtained from a polyethylenepolyamine reactor having a phosphorus-based catalyst. The feed typically contains AEEA.

Copending, commonly-assigned patent application entitled "Preparation Of Diethylenetriamine With Azeotrope Recycle", (D-14876), concurrently filed with this application, is incorporated herein by reference. Such application discloses processes for making ethyleneamines using a reductive amination or phosphorus-based catalyst in which a gaseous EDA/water phase is separated from the reactor effluent and at least a portion of the separated phase is returned to the reactor. In a preferred embodiment using a reductive amination catalyst, the recycle stream is admixed with MEA to break the azeotrope with water condensing out.

Copending, commonly-assigned patent application entitled "Water Addition To Enhance Phosphoric Acid Salt Catalyst Activity", (D-14877), concurrently filed with this application, is incorporated herein by reference. Such application discloses providing beneficial amounts of water in the reaction zone in which alkanolamine and another nitrogen compound are reacted to produce alky leneamines over a phosphorus-based catalyst. The water is believed to enhance or maintain or regenerate catalytic activity.

Copending, commonly-assigned patent application entitled "Use Of Pressure To Control The Noncyclics/-Cyclics Ratio Of Polyalkylene Polyamines", (D-14878), concurrently filed with this application, is incorporated herein by reference. Such application discloses processes for producing polyalkylenepolyamines over phosphorus-based catalysts in which control of the reation of noncyclic to cyclic products is achieved by controlling the level of the reaction pressure.

Another aspect of the invention is a process for separating triethylenediamine from a crude aqueous triethylenediamine reaction product mixture obtained from the preparation of triethylenediamine by the catalytic reaction of N-aminoethylpiperazine or N-hydroxyethylpiperazine. The triethylenediamine reaction product mixture also contains piperazine ammonia and hydrogen. The ammonia is removed from the triethylenediamine reaction, usually by use of a distillation (fractional) column and/or flash tank. A hydrocarbon extractant is added to the triethylenediamine reaction product mixture. Then an azeotrope of the piperazine and the hydrocarbon extractant is distilled from triethylenediamine reaction product mixture. A lower alkylene diol is added to the triethylenediamine reaction product mixture. Then an azeotrope of the triethylenediamine and the lower alkylene diol is distilled from triethylenediamine reaction product mixture. The unreacted N-aminoethylpiperazine or N-hydroxyethylpiperazine is removed from the triethylenediamine reaction product mixture, usually using a distillation column.

The hydrocarbon extractants can be those disclosed in U.S. Patent No. 3,105,019 (the pertinent parts of such patent are incorporated herein by reference). Nonane is the preferred hydrocarbon extractant.

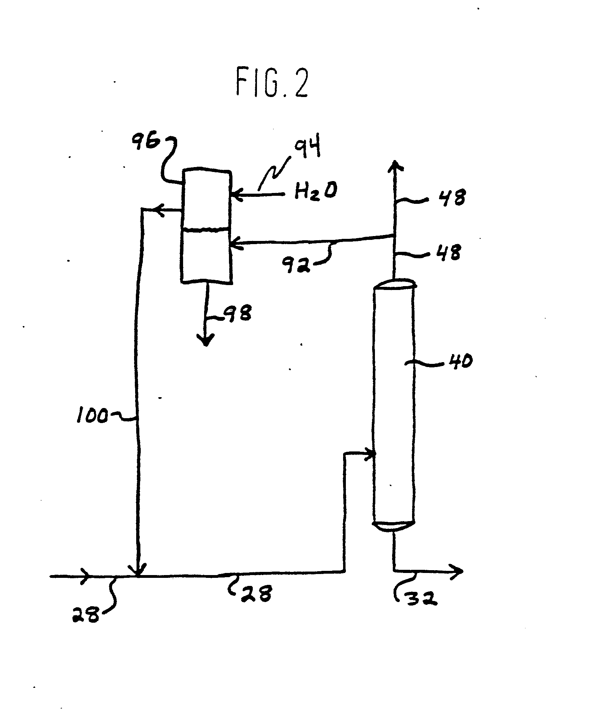

Referring to Figure 2, the azeotrope of nonane and piperazine comes off the top of column 40 and is sent via lines 48 and 92 to tank 96. (When the azeotrope boils at 133° to 136°C., it contains 43 weight percent of piperazine.) Warm water (line 94) is introduced into tank 96 in a countercurrent manner. An aqueous solution of piperazine is taken out of the bottom of tank 96 via line 98. (The composition is usually 35:65 of water: PIP.) The water breaks the nonane-piperazine azeotrope, so nonane is recycled to feed line 28 via line 100.

The lower alkylene diols can be those disclosed in U.S. Patent No. 4,233,447 (the pertinent parts of such patent are incorporated herein by reference). The lower alkylene diol preferably has the formula,

$$HO(\underset{R}{C}-\underset{R}{C})_nOH,$$

wherein n is 1 to 6 and R is H or lower alkyl ($C_1$-$C_6$), and most preferably is 1,4-butanediol.

**Claims**

1. Process for utilizing a reaction zone containing a catalyst to interchangeably conduct separate organic amine condensation reactions and a separation zone to interchangeably conduct separations of the reaction product streams of said reactions, comprising:

(a) conducting a first organic condensation reaction using a feed of a first reactant or set of reactants in said reaction zone in the presence of a catalytically effective amount of said catalyst and at a temperature and pressure sufficient for said first organic condensation reaction to occur;

(b) separating all or part of the reaction product stream from said reaction (a) in said separation zone into at least one constituent, said separation zone comprising at least two distillation columns;

(c) after termination of said first organic condensation reaction, conducting a second organic condensation reaction using a feed of a second reactant or set of reactants in said reaction zone (a) in the presence of a catalytically effective amount of said catalyst and at a temperature and pressure sufficient for said second organic condensation reaction to occur; and

(d) separating all or part of the reaction product stream from said reaction zone - (c), after termination of said separation - (b) of said first organic condensation reaction product stream (a), into at least one constituent.

2. The process as claimed in Claim 1 wherein said reaction is conducted in the vapor phase.

3. The process as claimed in Claim 1 wherein said reaction is conducted in the supercritical phase.

4. The process as claimed in Claim 1 wherein the liquid hourly space velocity of said reactants in said reaction is between about 0.1 and about 100 per hour.

5. The process as claimed in Claim 1 wherein said reaction feed (a) or said reaction feed (c) contains an alkyleneamine having at least two amino groups and an alkanolamine having at least one amino group and wherein a polyalkylene polyamine is produced in said reaction (a) or said reaction (c), respectively.

6. The process as claimed in Claim 5 wherein said alkyleneamine is ethylenediamine and said alkanolamine is monoethanolamine, and wherein the mole ratio of said ethylenediamine to said monoethanolamine is from 4:1 to 1:4.

7. The process as claimed in Claim 6 wherein said feed also contains ammonia.

8. The process as claimed in Claim 7 wherein said ammonia is present in an amount in excess of the molar amount participating in said reaction.

9. The process as claimed in Claim 5 wherein said reaction is conducted at a pressure of 200 to 2,000 p.s.i.g.

10. The process as claimed in Claim 1 wherein the feed also contains a gaseous diluent which is selected from the group consisting of nitrogen, argon, methane, water, helium, hydrogen and mixtures of at least two members of such group.

11. The process as claimed in Claim 1 wherein said reaction feed (a) or said reaction feed (c) contains a compound having the formula:

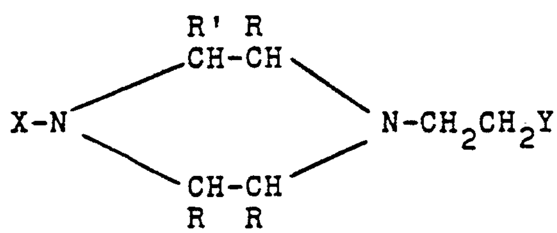

or a compound having the formula:

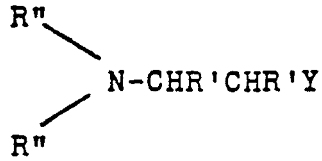

wherein R is selected from the group consisting of hydrogen, alkyl groups containing 1 to 12 carbon atoms and cycloalkyl groups containing 6 to 12 carbon atoms; R' is selected from the group consisting of hydrogen and alkyl groups containing 1 to 4 carbon atoms; R" is selected from the group consisting of hydrogen and -CHR'CHR'Y; X is selected from the group consisting of hydrogen and -$CH_2CH_2Y$; and Y is selected from the group consisting of -OH and -$NH_2$, and wherein diazabicyclo-(2.2.2)-octane or a C-substituted diazabicyclo-(2.2.2)-octane is produced in said reaction (a) or said reaction (c), respectively.

12. The process as claimed in Claim 11 wherein the reactant is N-aminoethylpiperazine or 2-hydroxyethylpiperazine and the product is diazabicyclo-(2.2.2)-octane.

13. The process as claimed in Claim 11 wherein said feed also contains ammonia and hydrogen.

14. The process as claimed in Claim 12 wherein said ammonia is present in an amount of excess of the molar amount participating in said reaction.

15. The process as claimed in Claim 11 wherein said reaction is conducted at a pressure of about one atmosphere absolute.

16. The process as claimed in Claim 1 wherein said reaction feed (a) or said reaction feed (c) contains ammonia and monoethanolamine, and wherein ethylenediamine is produced in said reaction (a) or said reaction (c), respectively.

17. The process as claimed in Claim 16 wherein said reaction is conducted at a pressure of 200 and 2,000 p.s.i.g.

18. The process as claimed in Claim 16 wherein said reaction feed of monoethanolamine and ammonia is prepared from ethylene oxide and ammonia.

19. The process as claimed in Claim 18 wherein said ammonia is present in a stoichiometrical excess.

20. The process as claimed in Claim 1 wherein said reaction feed (a) or said reaction feed (c) contains (i) an alkanol or an alkyl aldehyde and (ii) ammonia or a primary amine or a secondary amine, and wherein an alkyl amine is produced in said reaction (a) or said reaction (c), respectively.

21. The process as claimed in Claim 20 wherein said reaction is conducted at a pressure of 200 to 2,000 p.s.i.g.

22. The process as claimed in Claim 1 wherein said reaction feed (a) or said reaction feed (c) contains diglycolamine or a diglycolamine derivative, and wherein morpholine or a morpholine derivative, respectively, is produced in said reaction (a) or said reaction (c), respectively.

23. The process as claimed in Claim 22 wherein said reaction is conducted at a pressure of 200 to 2,000 p.s.i.g.

24. The process as claimed in Claim 1 wherein said catalyst is an amination catalyst.

25. The process as claimed in Claim 24 wherein said amination catalyst is a phosphorus acid or a phosphorus acid derivative compound.

26. The process of Claim 25 wherein the phosphoprus acid or acid derivative compound has the structure:

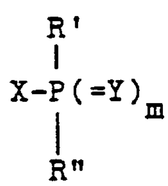

wherein Y is an oxygen or sulfur atom; m is 0 or 1; X is hydroxy, alkoxy, aryloxy, or the thioanalogs of the foregoing, alkyl or aryl substituted amino, halo, or the salts or phosphorus anhydrides or thioanhydrides of the foregoing when X is hydroxy or mercapto; R' and R" are each hydrogen, alkyl, aryl or one of the groups previously defined by X, R' and R" can be the same or different.

27. The process of Claim 26 wherein the phosphorus acid or acid derivative compound is phosphoric acid, phosphorus acid, boron phosphate, ferric phosphate, aluminum phosphate, hexaethylphosphorus triamide or hexamethylphosphorus triamide.

28. The process as claimed in Claim 25 wherein said catalyst is a phosphorus amide catalyst which has at least one phosphorus-nitrogen bond.

29. The process as claimed in Claim 28 wherein phosphorus amide catalyst has at least one P-N-C or P-N-H bond.

30. The process as claimed in Claim 28 wherein the phosphorus amide catalyst is a phosphoramidous, phosphordiamidous, phosphoramidic, phosphordiamidic, phosphonamidous, or phosphonamidic acid, ester, half-ester, anhydride, or metal salt, a triamino phosphine or triaminophosphine oxide, a phosphonic or phosphonous diamide, a phosphinic amide, or a phosphonic amide.

31. The process as claimed in Claim 25 wherein said catalyst is a metal phosphate catalyst.

32. The process as claimed in Claim 31 wherein said metal phosphate catalyst is a metal acid phosphate catalyst.

33. The process as claimed in Claim 31 wherein said metal acid phosphate catalyst is a solid, insoluble, metal acid phosphate catalyst.

34. The process as claimed in Claim 31 wherein said metal acid phosphate is a Group IIIB metal acid phosphate, a Group IIIB metal monohydrogen phosphate or a Group IIIB dihydrogen phosphate, and wherein said Group IIIB metal is scandium, yttrium, lanthanum or a rare earth lanthanide having an atomic number from 58 to 71.

35. The process as claimed in Claim 31 wherein said metal acid phosphate catalyst is a Group IIA metal acid phosphate catalyst or a Group IVB metal acid phosphate catalyst.

36. The process as claimed in Claim 25 wherein said phosphorus-containing catalyst is supported on a carrier.

37. The process as claimed in Claim 25 wherein said phosphorus-containing catalyst which is supported on carrier selected from the group consisting of alumina, silica, silica-alumina, diatomaceous earth, silica-titania and mixtures of at least two members of such group.

38. The method as claimed in Claim 24 wherein said amination catalyst is a reductive amination catalyst.

39. The method as claimed in Claim 38 wherein said reductive amination catalyst is comprised of nickel impregnated on a conventional catalyst support material.

40. The method as claimed in Claim 38 wherein said reductive amination catalyst is comprised of nickel impregnated on a support material selected from the group consisting of alumina, silica, silica-alumina, diatomaceous earth, silica-titania and mixtures of at least two members of such group.

41. The method as claimed in Claim 40 wherein small perc ntages of rhenium and boron are present in said nickel.

42. The method as claimed in Claim 38 wherein said reductive amination catalyst is a nickel, cobalt or rhodium catalyst, wherein the metal is present on the surface of the catalyst in a polyatomic form.

43. The method as claimed in Claim 42 wherein said catalyst is Raney nickel or Raney copper.

44. The process as claimed in Claim 1 wherein said at least one constituent separated from said reaction stream (a) is an organic amine condensation product and wherein said at least one constituent separated from said reaction stream (c) is a different organic amine condensation product.

45. The process as claimed in Claim 44 wherein said reaction feed for said reaction - (a) also contains a gaseous diluent, and wherein, at the start of said separation step - (a), part of all of said diluent is flashed off from said reaction product stream (a).

46. The process as claimed in Claim 44 wherein said reaction feed for said reaction - (a) also contains ammonia and wherein, at the start of said separation step (b), all or part of said unreacted ammonia is flashed off from

said reaction product stream (a).

47. The process as claimed in Claim 44 wherein said separation (b) of the at least one constituent of from said reaction product stream (a) is achieved using at least four distillation columns.

48. The process as claimed in Claim 47 wherein five of said distillation columns are used to effect said separation.

49. The process as claimed in Claim 48 wherein said distillation columns are fractional distillation columns.

50. The process as claimed in Claim 49 wherein water is removed from said reaction product stream (a) by means of said second distillation column.

51. The process as claimed in Claim 49 wherein at least part of the unreacted reactants in said reaction product stream (a) are removed and are each recycled in part or all to said reaction zone during said reaction (a).

52. The process as claimed in Claim 44 wherein said reaction feed for said reaction - (c) also contains a gaseous, inert diluent, and wherein, at the start of said separation step - (d), part or all of said diluent is flashed off from said reaction product stream (c).

53. The process as claimed in Claim 44 wherein said reaction feed for said reaction - (c) also contains ammonia, and wherein, at the start of said separation step (d), all or part of said unreacted ammonia is flashed off from said reaction product (c).

54. The process as claimed in Claim 44 wherein said separation (d) of the at least one constituent of from said reaction product stream (c) is achieved using at least four distillation column.

55. The process as claimed in Claim 54 wherein five of said distillation columns are used to effect said separation.

56. The process as claimed in Claim 55 wherein said distillation columns are fractional distillation columns.

57. The process as claimed in Claim 56 wherein water is removed from said reaction product stream (c) by means of said first distillation column.

58. The process as claimed in Claim 56 wherein the unreacted reactants in said reaction product stream (c) are removed and are each recycled in part or all to said reaction zone during said reaction (c).

59. Process for utilizing a separation zone to interchangeably conduct separations of separate reaction product streams from separate organic amine condensation reactions, comprising:

(a) conducting a first separation in said separation zone of a first reaction product stream of a first organic amine condensation reaction into at least one constituent said reaction zone comprising at least two distillation columns; and

(b) after termination of said first separation, conducting a second separation in said separation zone of a second reaction product stream of a second organic amine condensation reaction into at least one constituent.

60. The process as claimed in Claim 59 wherein said separation zone comprises five fractional distillation columns.

61. The process as claimed in Claim 60 wherein said first reaction product stream and said second reaction product stream are each at least one of the following: a morpholine reaction product stream, a diethylenetriamine reaction product stream, a triethylenediamine reaction product stream, a butylamines reaction product stream and an ethylenediamine reaction product stream.

62. Process for separating the triethylenediamine from a crude triethylenediamine reaction product mixture obtained from the preparation of triethylenediamine by the catalytic reaction of N-aminoethylpiperazine or N-hydroxyethylpiperazine, said triethylenediamine reaction product mixture also containing piperazine ammonia and hydrogen,comprising;

(a) removing said ammonia from said triethylenediamine reaction product mixture;

(b) removing said hydrogen from said triethylenediamine reaction product mixture of step (a);

(c) adding a hydrocarbon extractant to said triethylenediamine reaction product mixture of step (b);

(d) distilling an azeotrope of said piperazine and said hydrocarbon extractant from said triethylenediamine reaction product mixture of step (c);

(e) adding a lower alkylene diol to said triethylenediamine reaction product mixture of step (d);

(f) distilling an azeotrope of said triethylenediamine and said lower alkylene diol from triethylenediamine reaction product of step (e); and

(g) removing said unreacted N-aminoethylpiperazine or said N-hydroxyethylpiperazine from said triethylenediamine reaction product mixture of step (f).

63. The process as claimed in Claim 62 wherein said steps (a), (b) and (g) are conducted in distillation columns.

64. The process as claimed in Claim 62 wherein said hydrocarbon extracted in step - (d) is nonane.

65. The process as claimed in Claim 62 wherein said lower alkylene diol in steps (e) and (f) has the formula:

$$HO(\underset{R}{\underset{|}{C}}-\underset{R}{\underset{|}{C}})_nOH,$$

wherein n is 1 to 6 and R is H or lower alkyl.

66. The process as claimed in Claim 65 wherein said lower alkylene diol is 1,4-butanediol.

67. The process as claimed in Claim 62 wherein said azeotrope of said piperazine and said hydrocarbon extractant from step (d) is treated with water to form separated hydrocarbon extractant and an aqueous solution of piperazine, at least part of said separated hydrocarbon extractant being recycled to step - (c).

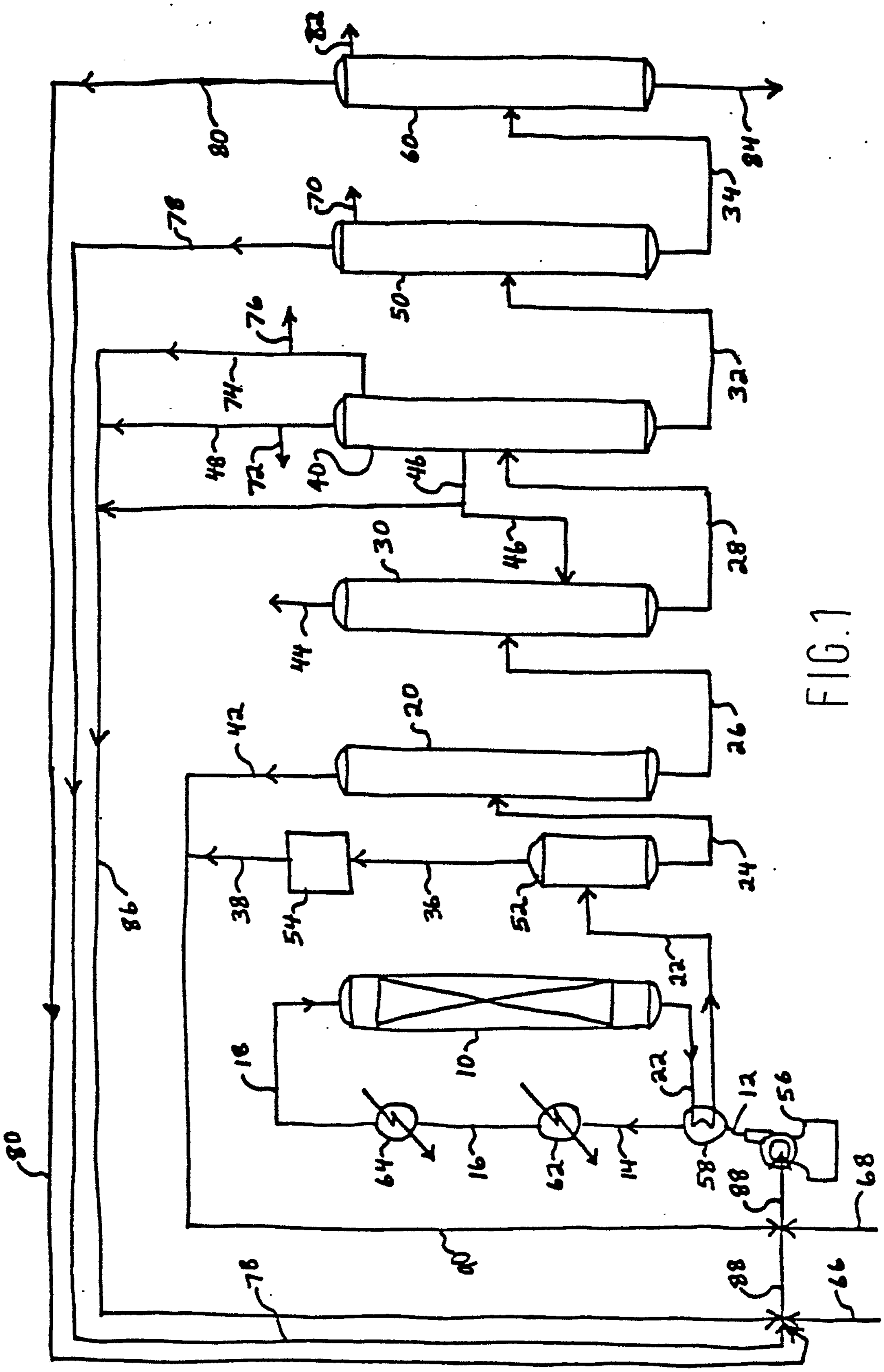

FIG. 1

FIG. 2